# EUROPEAN PATENT APPLICATION

(11) **EP 2 719 757 A1**
(43) Date of publication of application: **16.04.2014**
(21) Application number: 13199167.1
(22) Date of filing: 03.09.2010
(51) Int. Cl.: C12N 1/21, C12N 15/31, C12N 15/52, C12P 19/02

(54) **Production of secreted bioproducts from photosynthetic microbes**

(30) Priority: 04.09.2009 US 239985 P; 24.03.2010 US 317001 P
(62) Divisional of application: 10814563.2
(71) Applicant: President and Fellows of Harvard College, Cambridge, MA 02138 (US)
(72) Inventor: Niederholtmeyer, Henrike, 49078 Osnabruck (DE); Way, Jeffrey Charles, Cambridge, Massachusetts 02138 (US); Wolfstaedter, Bernd, 74193 Schwaigern (DE); Savage, David, Cambridge, Massachusetts 02139 (US)
(74) Representative: Sweetinburgh, Mark Roger

(57) **Abstract**

The invention provides new compositions and methods for the production of useful chemicals from recombinant photosynthetic bacteria, such as recombinant cyanobacteria. Embodiments of the invention provide for the genetic engineering of photosynthetic bacteria to express and secrete a product of interest, such as hexose sugars or lactate. More specifically, the recombinant cyanobacteria express heterologous genes for both product expression and secretion. In some embodiments, the recombinant cyanobacteria express heterologous genes for secretion of products coupled to a pH gradient across a cell membrane. Sugars, such as sucrose, glucose and fructose, and other useful chemicals, such as lactic acid, are produced via the invention.

## Description

### CROSS-REFERENCE TO RELATED APPLICATIONS

This application claims the benefit of priority under 35 U.S.C. § 119(e) of U.S. Provisional Application Nos. 61/317,001, filed March 24, 2010 and 61/239,985 filed September 4, 2009, the contents of each of which are incorporated herein by reference in its entirety.

### FIELD OF THE INVENTION

The invention relates to compositions and methods for the production of useful chemicals from photosynthetic microbes.

### BACKGROUND

The cost of commodity chemicals limits the growth of the world economy. Currently chemicals, such as sugars, are made from green plants with limited geographical distribution, while other natural products such as lactic acid and succinic acid are made by metabolic engineering using sugars as a starting material. These carbon-based molecules are ultimately derived from carbon dioxide fixation, but the existing processes are inefficient in many ways. For example, in the production of sugar by green plants, most of the fixed carbon goes into the biomass of the plant, such as cellulosic biomass that is relatively resistant to further breakdown.

Ideally, photosynthetic microbes could directly produce such molecules, much as ethanol is produced by yeast, because of the efficient use of light energy by these organisms and the potential for CO₂ mitigation during production. Currently, most photosynthetic bacteria do not secrete carbon-based molecules, likely because of the metabolic cost of their synthesis. In addition, and unlike ethanol, many hydrophilic molecules such as glucose, lactic acid and succinic acid cannot pass through cell membranes under normal conditions. Therefore, there is a need in the art for methods and systems that allow the production and secretion of hydrophilic molecules from photosynthetic bacteria.

### SUMMARY

The present invention provides compositions and methods for the production of useful chemicals from photosynthetic bacteria, such as cyanobacteria.

In one embodiment, the invention provides genetically engineered photosynthetic cyanobacteria that express at least one heterologous enzyme and at least one heterologous transporter, wherein the transporter mediates the secretion of a product into an extracellular medium. For example, the transporter mediates the secretion of a product whose synthesis is enhanced by the heterologous enzyme. In a particular embodiment, the heterologous enzyme is an invertase, such as *invA*; and the transporter is *glf.* In another embodiment, the heterologous enzyme is a dehydrogenase, such as *ldhA,* and the transporter is *lldP.* The cyanobacteria may further include a resistance marker.

In some embodiments, the heterologous transporter is a member of the maj or facilitator superfamily, including the Glut family of glucose transport proteins, the HXT family of glucose transporters, *lacY, lldP,* a mammalian citric acid transport protein, the *glpT* glycerol phosphate transporter, a sucrose transporter, an amino acid transporter, a glutamate transporter, and *GLF.* In some embodiments, the heterologous hexose (e.g., glucose) transporters can be the Glut, HXT, and GLF proteins.

In further embodiments, the recombinant photosynthetic microbes further comprise additional heterologous genes that encode enzymes for enhancing synthesis of intracellular precursors. In a particular embodiment, the additional heterologous gene is *udhA,* which encodes NAD(P)H transhydrogenase. In specific embodiment, the additional heterologous gene is *galU,* which encodes UDP-glucose phosphorylase. In alternative embodiments, the additional heterologous gene encodes an enzyme for sucrose synthesis, such as sucrose phosphate synthase and sucrose phosphate phosphatase.

In yet further embodiments, the recombinant microbe further comprises at least one reduction-of function mutation that can enhance production of hydrophilic molecules. In some embodiments, the reduction-of-function mutation is a deletion in at least one endogenous kinase gene that phosphorylates a hydrophilic product. In a particular embodiment, the endogenous kinase is hexokinase. In another embodiment, the endogenous kinase is glucokinase. In a particular embodiment, the reduction-of-function mutation is a deletion in genes encoding any membrane-associated NAD(P) transhydrogenase.

In some embodiments, genetically engineered photosynthetic microbes are cyanobacteria. In some embodiments, the transporter is a member of the major facilitator superfamily. In some embodiments, the compound is a chiral compound. Examples of chiral compounds include, without limitation, sugars and carboxylic acids, such as lactic acid. Examples of sugars include glucose, fructose, a mixture of glucose and fructose, or sucrose.

In some embodiments, the enzyme is expressed from an inducible promoter. Non-limiting examples of inducible promoters include a *lac* operon promoter, a nitrogen-sensitive promoter, and a salt-inducible promoter. In one embodiment, the inducible promoter is IPTG-inducible promoter. In another embodiment, the inducible promoter is NaCl-inducible promoter.

In another aspect, the invention provides for a recombinant photosynthetic bacterium, such as cyanobacterium, that expresses at least one heterologous transporter that is coupled to the pH gradient across a cell membrane. In some embodiments, the invention provides for a recombinant photosynthetic bacterium, such as a cyanobacterium, that expresses at least one heterologous transporter that co-transports a proton with a product (e.g., a metabolite). In alternative embodiments, the invention provides genetically engineered photosynthetic bacteria, such as cyanobacteria, that express a heterologous transporter that co-transports a sodium ion with a product (e.g., a metabolite).

In some embodiments, the heterologous transporter mediates the symport or antiport of a product and an inorganic ion. Examples of an inorganic ion include a proton, a sodium ion, a potassium ion, a chloride ion, and a hydroxide ion.

In some embodiments, the heterologous transporter can be a lactic acid/proton symporter, a sucrose proton symporter, a lactose/proton symporter, a dicarboxylic acid/proton symporter, an amino acid/proton symporter, a citrate/proton symporter, an amino acid/sodium ion symporter, or an amino acid/hydroxide ion antiporter. Examples of such heterologous transporters include, but not limited to, a sucrose transporter CscB, a lactose permease *of E. coli* encoded by the *lacY* gene, a mammalian H⁺/peptide transporter PepT1H, a glutamate transporter GltS, and a lactate transporter *lldP.*

In yet a another aspect, the invention provides a method for producing a hydrophilic product, the method comprising culturing a recombinant cyanobacterium according to the invention in the presence of light and CO₂, and obtaining the product from a supernatant of the cultured microbe. In some aspects, the method further includes providing an inducing agent to induce the expression of at least one heterologous gene expressed in the recombinant bacterium. In one embodiment, the heterologous gene encodes an enzyme, a transporter or both. In particular embodiments, the hydrophilic product is glucose and/or fructose, or lactic acid.

In yet another aspect, the invention provides a method for producing a hydrophilic product, the method comprising culturing a cyanobacteria according to the second aspect of the invention in the presence of light and CO₂, and obtaining said product from a supernatant of the cultured microbe. In some aspects the method further includes providing an inducing agent to induce the expression of at least one heterologous gene expressed in cyanobacteria. In one embodiment, the heterologous gene encodes an enzyme, a transporter or both. In a particular embodiment, the hydrophilic product is lactic acid, sucrose, an amino acid, citric acid, malic acid, fumaric acid, or succinic acid.

### DESCRIPTION OF THE DRAWINGS

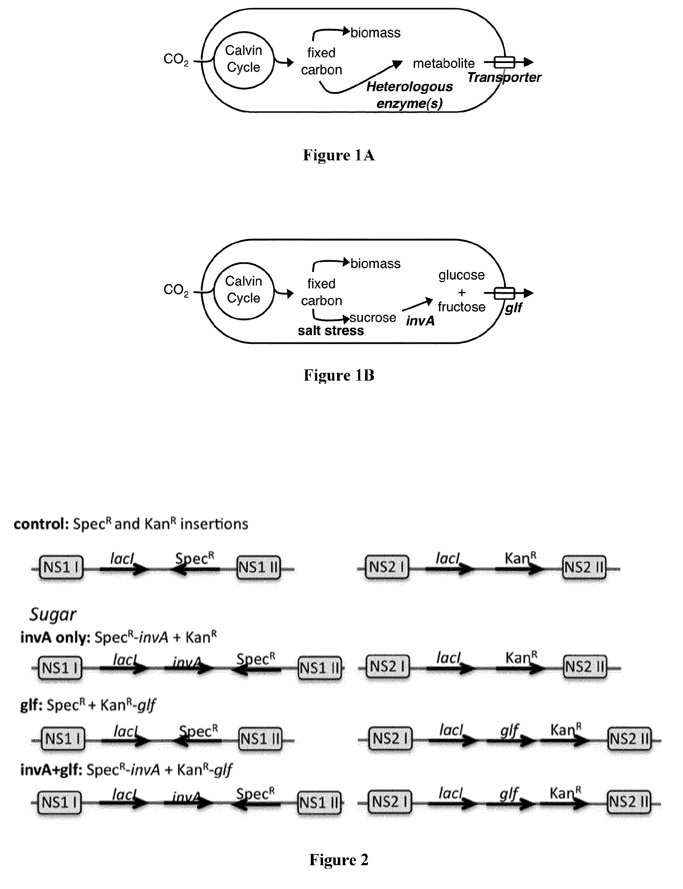

Figures 1A and 1B show schematic diagrams of engineering strategies for production of bioproducts by a genetically engineered cyanobacterium. Figure 1A illustrates a cyanobacterial cell in which one or more heterologous enzymes have been expressed to produce a hydrophilic metabolite that cannot spontaneously cross a cell membrane, and in which a heterologous transporter has been introduced to allow secretion of the metabolite from the cell. Figure 1B shows an engineering scheme for production of hexose sugars (e.g., glucose and fructose) by a genetically engineered *Synechococcus elongatus* 7942. *Synechococcus* naturally produces sucrose in response to salt stress by fixing carbon via natural pathways such as Calvin Cycle. The bacteria are engineered to express invertase (encoded by *invA*), which cleaves sucrose into glucose and fructose, and the *glf* gene encoding a glucose- and fructose-facilitated diffusion transporter, which allows export of the sugars from the cell. Color versions of drawings are available in Niederholtmeyer et al., 76 Appl. Environ. Microbio. 3462 (2010).

Figure 2 shows plasmid vectors that mediate integration of the *invA* and *glf* genes into the *S. elongatus* 7942 genome by homologous recombination. Derivatives of *S*. *elongatus* were constructed by insertion of transgenes into "neutral sites" NS1 and NS2 with spectinomycin-resistance and kanamycin-resistance markers, respectively. Strains lacking a particular transgene were transformed with empty spectinomycin-resistant and kanamycin-resistant vectors as controls.

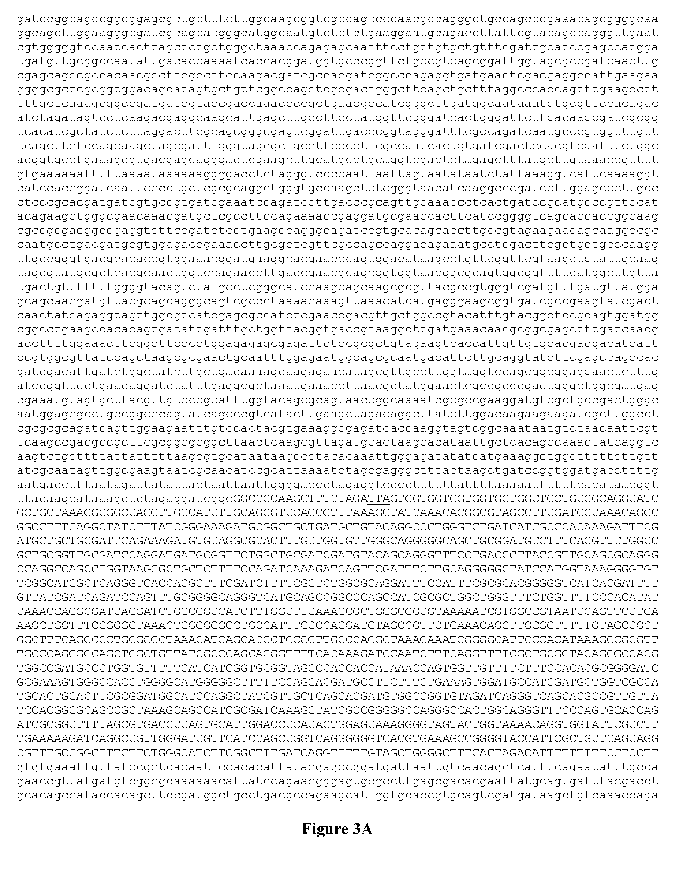

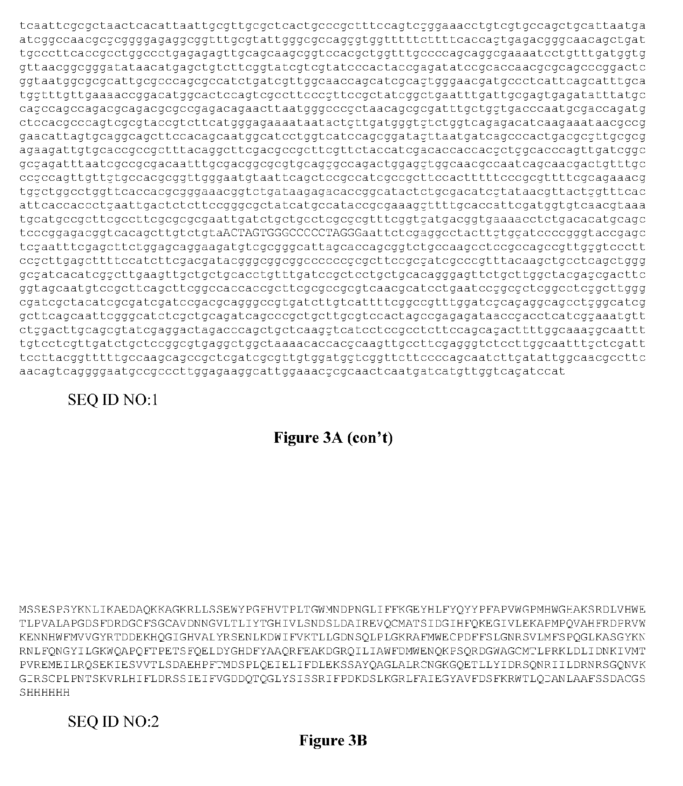

Figures 3A and 3B show the gene and protein sequences of the inserted *invA* into *S. elongatus* 7942. Figure 3A shows the sequence of *invA*, spectinomycin-resistance, and *lacI* genes inserted into NS1 of *S*. *elongatus* 7942. The region encoding the *invA* gene plus immediately flanking regions is shown in capital letters. This gene is encoded in the antisense orientation. The start and stop codons are underlined. The plasmid carrying the *invA* gene is termed DS1321. Figure 3B shows the sequence of the invertase protein, including a His₆ tag at the C-terminus, as expressed in *S. elongatus* 7942.

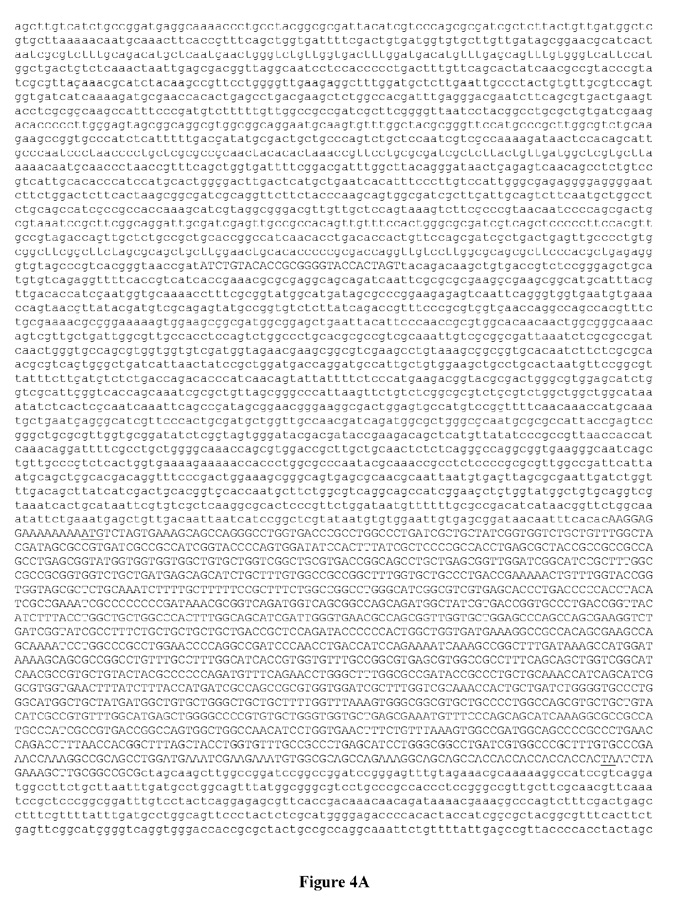

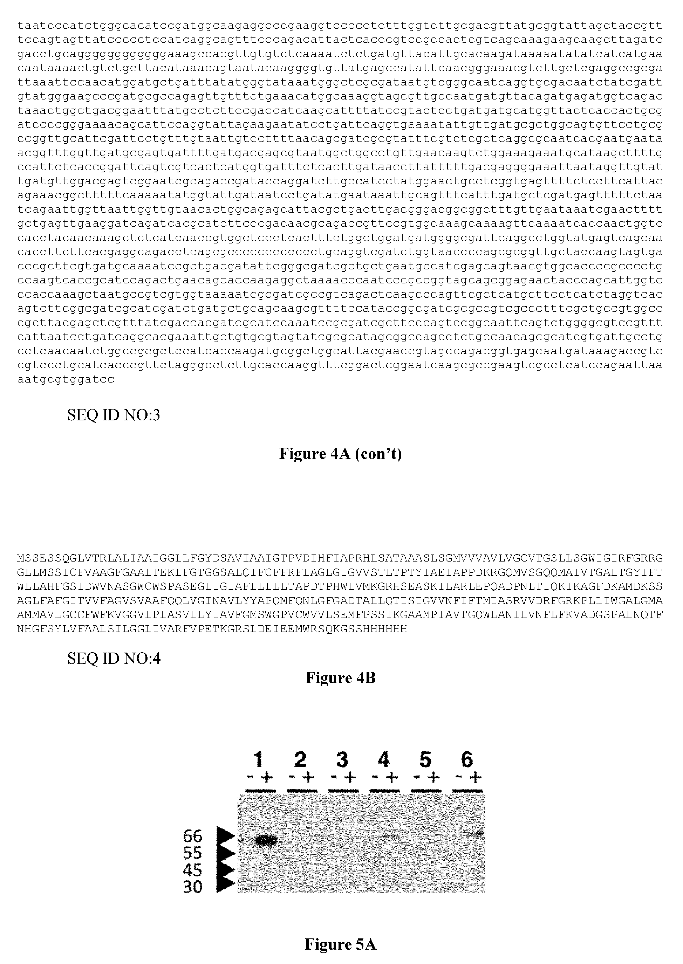

Figures 4A and 4B show the gene and protein sequences of the inserted *glf* into *S. elongates* 7942. Figure 4A shows the sequence of *glf*, kanamycin-resistance, and *lacI* genes inserted into NS2 of *S*. *elongatus* 7942. The region encoding *glf* and immediately flanking sequences are shown in capitals, with the start and stop codons underlined. The plasmid carrying the *glf* gene is termed DS21. Figure 4B shows the sequence of the *GLF* protein, including a His6 tag at the C-terminus, as expressed in S. *elongatus* 7942.

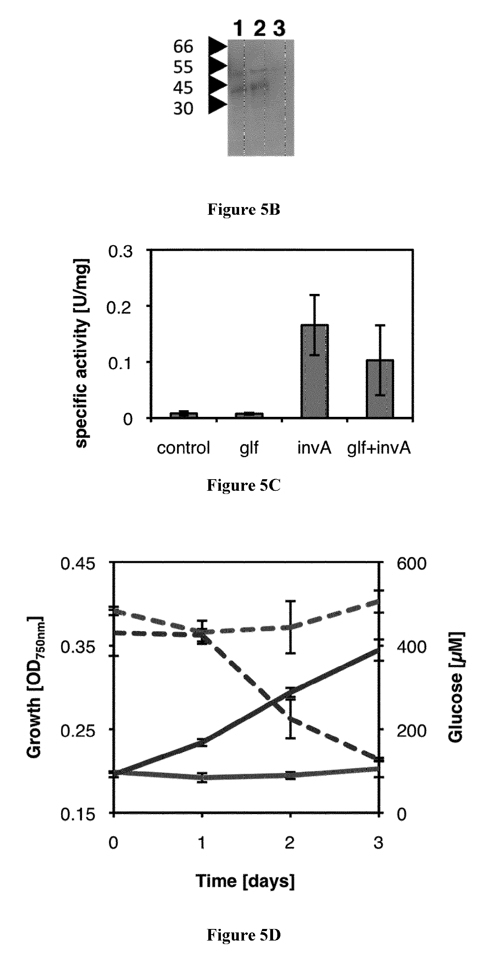

Figures 5A to 5D show the expression of transgenes to promote hexose sugar or lactate synthesis and secretion. Figure 5A shows the western blot assay for expression of His₆-tagged *invA* and *glf* in *E*. *coli* and *Synechococcus.* Predicted molecular weights of tagged *InvA* and *GLF* are 60,000 and 55,400 respectively. Symbols '-' and '+' indicate samples growth without or with 1 mM IPTG. Lane 1 - *E. coli, invA* expression plasmid; lane 2 - *E. coli, glf* expression plasmid; lane 3 - *S*. *elongatus,* no plasmid; lane 4 - *S*. *elongatus, invA* expression plasmid; lane 5 - *S*. *elongatus, glf* expression plasmid; lane 6 - *S*. *elongatus, invA* and *glf* expression plasmids. (The *glf* product was not detected.) Figure 5B shows a western blot assay for expression of His₆-tagged *ldhA* and *lldP* in *Synechococcus* containing the *ldhA* and *lldP* expression constructs. Lanes 1 to 3: 100 mM, 1 mM, and 0 IPTG, respectively. (The *lldP* product was not detected.) Figure 5C shows invertase enzyme activity from extracts of *Synechococcus* expressing *invA, glf,* or both transgenes. Figure 5D shows functional expression of the *glf* transporter in *Synechococcus*, assayed by growth of *Synechococcus* in the dark. Solid lines: growth; dotted lines: disappearance of glucose from culture medium. The lines that remain fairly constant correspond to wild-type *S. elongatus* 7942; the decreasing dotted line and increasing solid line correspond to recombinant *S. elongatus* 7942 that expresses the *glf* gene.

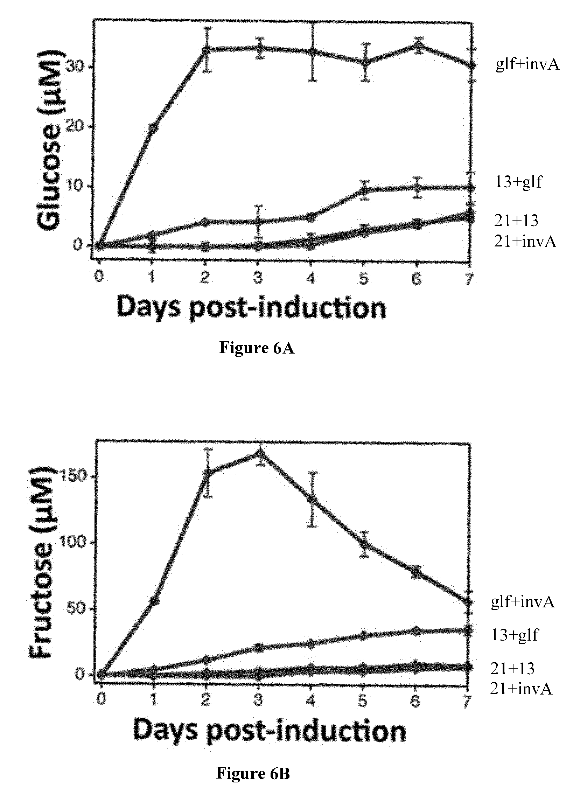

Figures 6A and 6B show sugar production in culture medium of *Synechococcus* strains expressing *Z*. *mobilis glf* and *invA* (cross), only *glf* (triangle), only *invA* (square), or neither transgene (diamond). The x axis shows the time after induction of sucrose synthesis with 200 mM NaCl and of transgenes with 100 µM IPTG. Symbol "21" refers to plasmid DS21; "13" refers to plasmid DS1321. Figure 6A shows extracellular concentration of glucose of the four strains. Figure 6B shows extracellular concentration of fructose of the four strains.

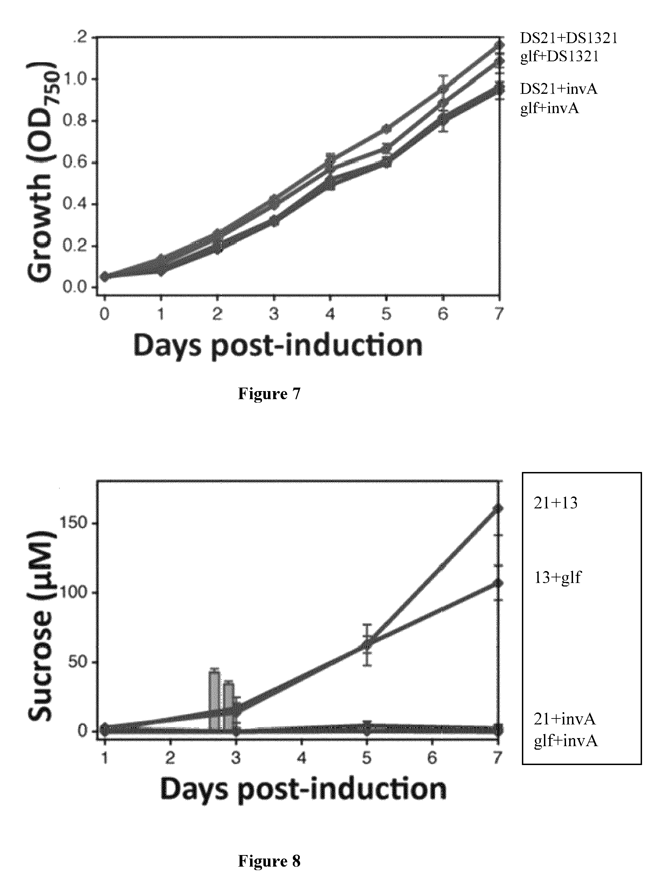

Figures 7 shows the growth curves of cyanobacteria engineered to express *invA* and *glf* from *Z*. *mobilis* (square), only *glf* (cross), only *invA* (triangle), or neither transgene (diamond). The x axis shows the time after induction of sucrose synthesis with 200 mM NaCl and of transgenes with 100 µM IPTG.

Figures 8 shows the extracellular concentration of sucrose produced by *Synechococcus* strains expressing *Z*. *mobilis glf* and *invA* (glf+invA), only *glf* (13+glf), only *invA* (21+invA), or neither transgene (21+13). The bars show the concentrations of both intracellular and extracellular sucrose determined from a Day-3 whole culture sample (cells and culture supernatant); less than 3 µM sucrose was detected in the *invA*-expressing strains.

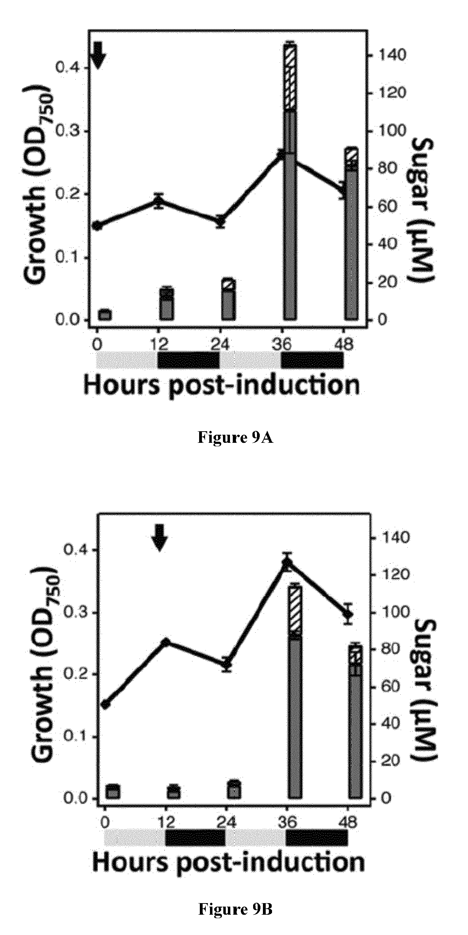

Figures 9A and 9B show growth and sugar production or consumption as a function of a cycle consisting of 12-h days and 12-h nights. The lines indicate OD₇₅₀; bars indicate the concentrations of extracellular fructose (gray bars) and glucose (striped bars). The arrows indicate the induction with NaCl and IPTG at dawn (Figure 9A) and at dusk (Figure 9B).

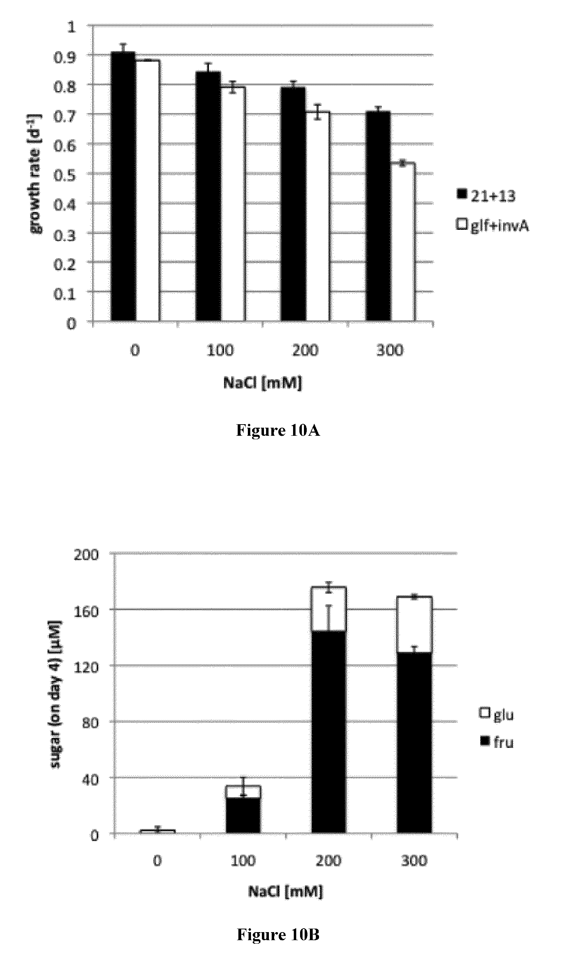

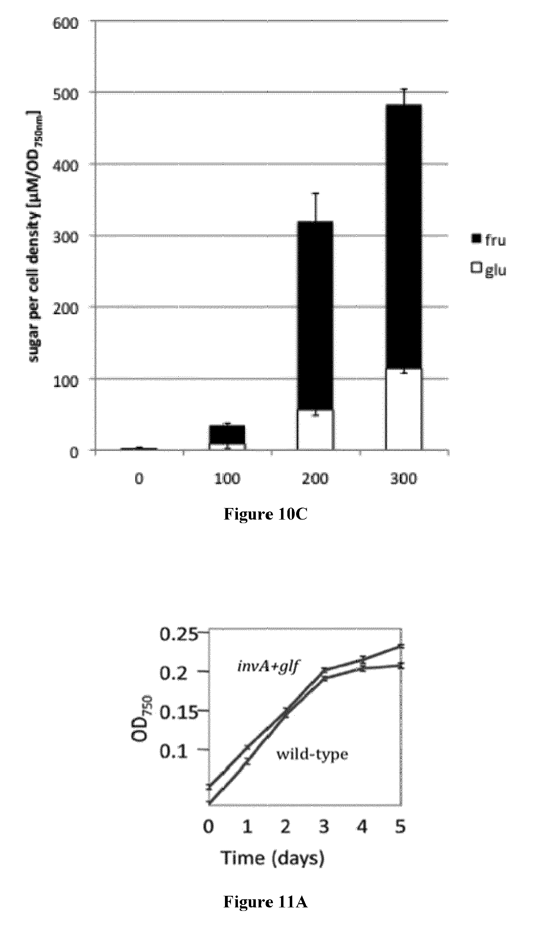

Figures10A to 10C show the growth and sugar production of *glf*+*invA*-expressing and control cells as a function of NaCl concentration. Figure 10A shows the growth rates of a *Synechococcus* empty vector control. Symbol "21+13" and *glf*+*invA* strain after induction with 100 mM IPTG and various different NaCl concentrations in BG-11 medium. Figure 10B shows the concentrations of glucose and fructose in the culture medium of a *Synechococcus glf*+*invA* strain four days after induction with 100 mM IPTG and various different NaCl concentrations in BG-11 medium. Figure 10C shows the extracellular concentrations of glucose and fructose produced by a *Synechococcus glf*+*invA* strain on a per cell basis.

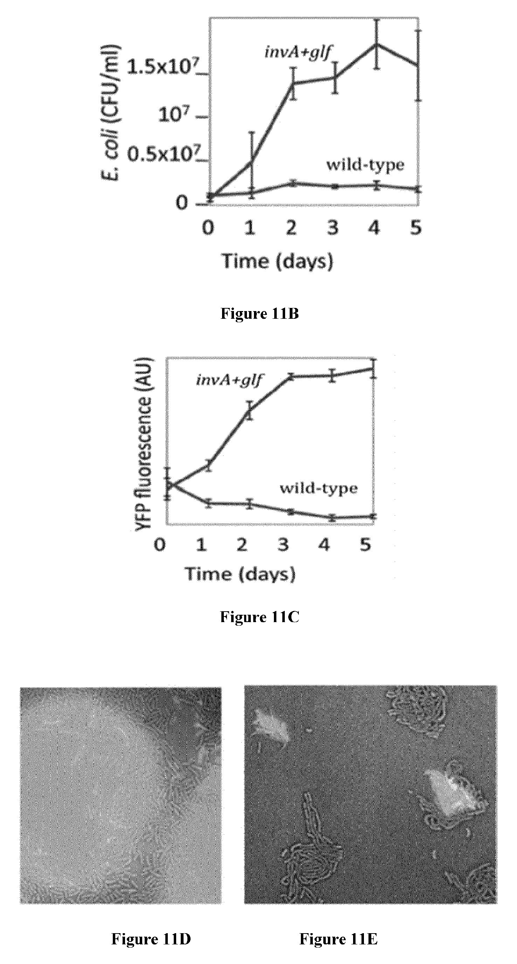

Figures 11A to 11E show that sugar-secreting *Synechococcus* supports *E. coli* growth in coculture. For Figures 11A to 11C, *E. coli* DH5α containing a YFP expression plasmid was diluted to obtain a concentration of 10⁶ cells/ml in wild-type *Synechococcus* cultures or *Synechococcus* cultures expressing *glf* and *invA* in BG-11 medium with 200 mM NaCl, 1 mg/ml NH₄Cl, and appropriate antibiotics. Figure 11A shows continued growth of engineered and wild-type *Synechococcus* in the presence of *E*. *coli.* Figures 11B to 11C show growth of *E*. *coli* in the presence of engineered or wild-type *Synechococcus* strains, as determined by the number of CFU (Figure 11B) or YFP fluorescence (Figure 11C). Figures 11D to 11E show coculture of sugar-secreting *Synechococcus* and *E. coli* on agar plates at two different NaCl concentrations. *E. coli* YFP fluorescence (lighter shade); *Synechococcus* chlorophyll auto fluorescence (coiled features). Microcolonies formed on BG-11 agar with 100 mM NaCl (Figure 11D) and 200 mM NaCl (Figure 11E) four days after plating.

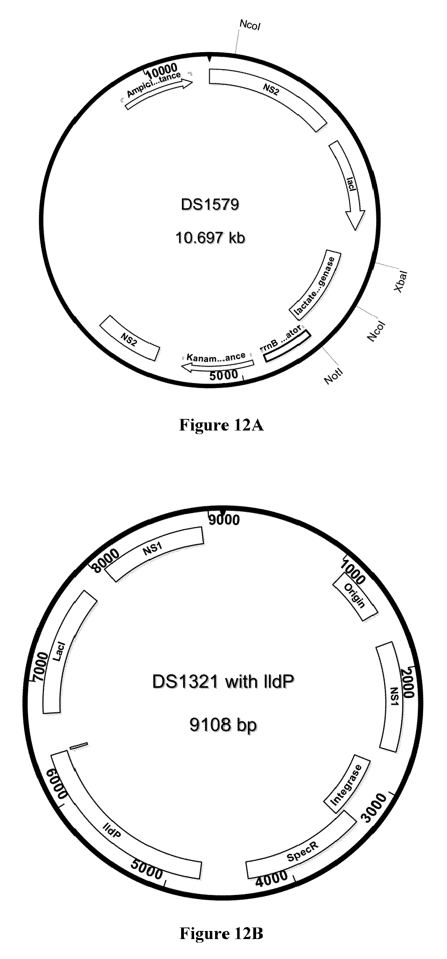

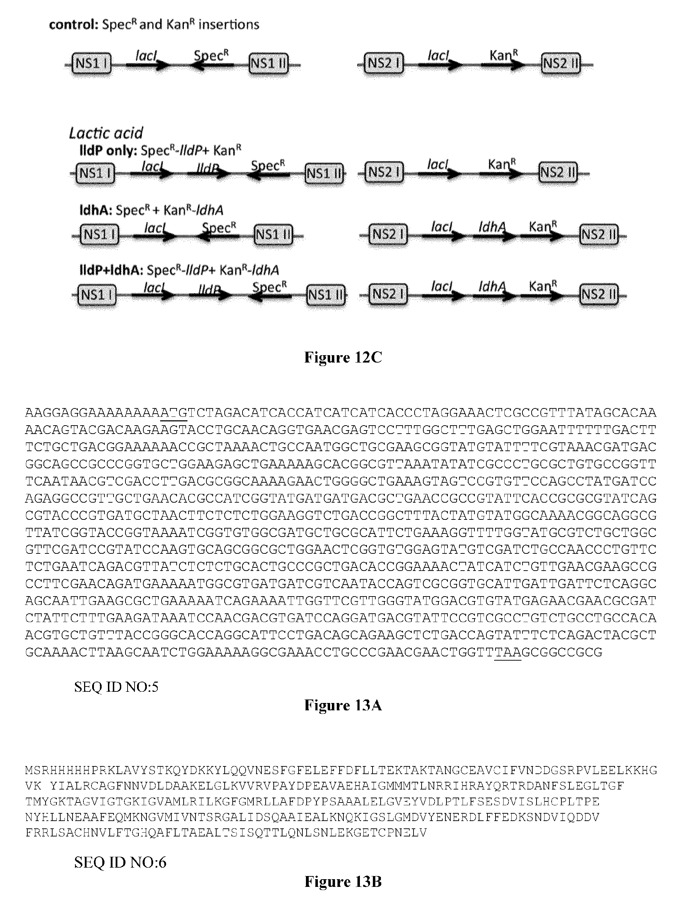

Figures 12A to 12C show plasmid schematics for expression of *ldhA* and *lldP* in *S. elongatus* 7942. Figure 12A shows the plasmid for expression of *ldhA.* Figure 12B shows the plasmid for expression of *lldP.* Figure 12C shows construction of derivatives of *S. elongatus* by insertion of *lldP* and *ldhA* genes into "neutral sites" NS 1 and NS2 with spectinomycin-resistance and kanamycin-resistance markers, respectively. Strains lacking a particular transgene were transformed with empty spectinomycin-resistant and kanamycin-resistant vectors as controls.

Figures 13A to 13B show the sequence of the inserted *ldhA* gene and expressed protein in *S. elongatus* 7942. Figure 13A shows the nucleotide sequence of the inserted *ldhA* gene in *S. elongatus.* Start and stop codons are underlined. Figure 13B shows the amino acid sequence of *ldhA* gene encoding lactate dehydrogenase, including a His₆ tag at the N-terminus, as expressed in *S. elongatus* 7942.

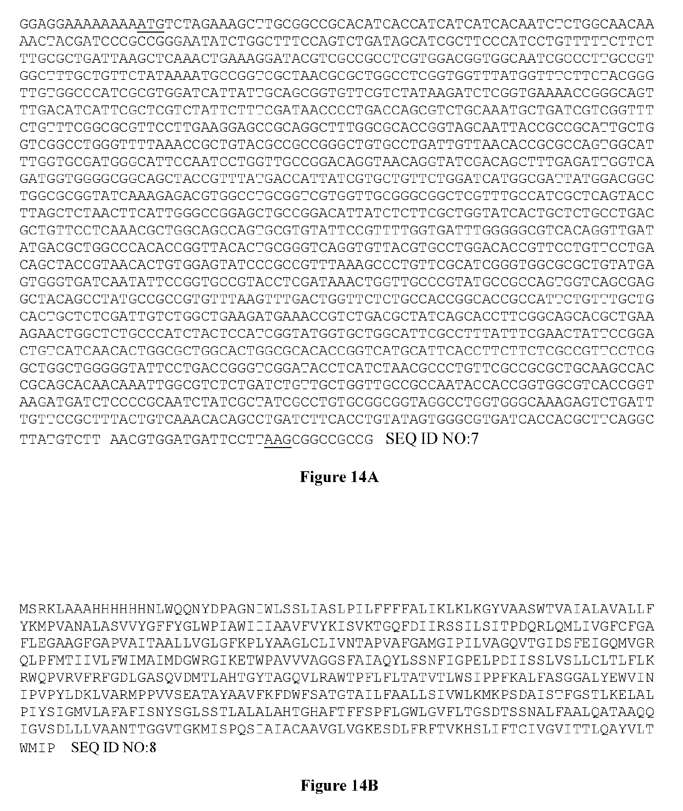

Figures 14A and 14B show the sequence of the inserted *lldP* gene and expressed protein in *S. elongatus* 7942. Figure 14A shows the nucleotide sequence of the inserted *lldP* gene in *S. elongatus.* Start and stop codons are underlined. Figure 14B shows the amino acid sequence of the expressed protein encoded by *lldP,* including a His₆ tag at the N-terminus, as expressed in *S. elongatus* 7942.

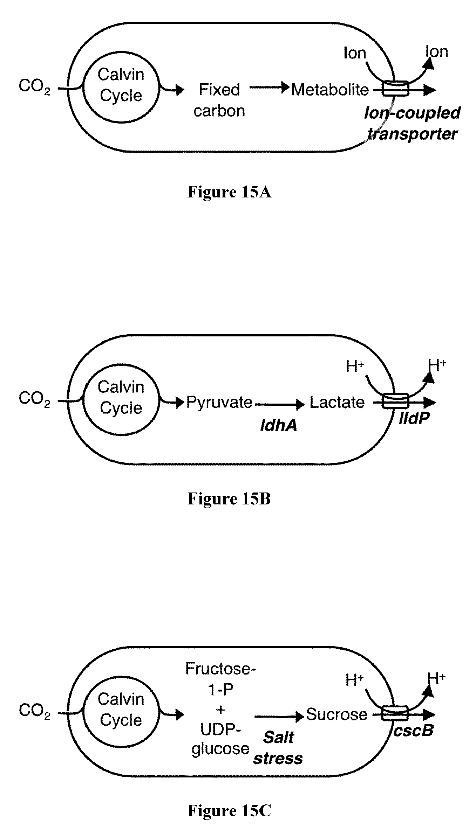

Figures 15A to 15C show schematic diagrams for production of bioproducts by a recombinant cyanobacterium that expresses an ion-coupled transporter. Figure 15A illustrates a cyanobacterial cell in which an ion-coupled transporter is expressed to allow secretion of the product from the cell in a manner that is assisted by the concentration gradient of the ion. Figure 15B shows the engineering scheme for cyanobacterial production of lactic acid (lactate). *Synechococcus* naturally produces pyruvate as a metabolic intermediate by fixing carbon via Calvin cycle. The bacteria are engineered to express both lactate dehydrogenase (*ldhA*), which produces lactic acid, and a transporter (*lldP*), which cotransports lactate and H⁺ from the cell. Figure 15C shows the engineering scheme for cyanobacterial production of sucrose. *Synechococcus* naturally produces intracellular sucrose in response to salt stress. The bacteria are engineered to express the *cscB* gene, which encodes a proton-coupled sucrose transporter.

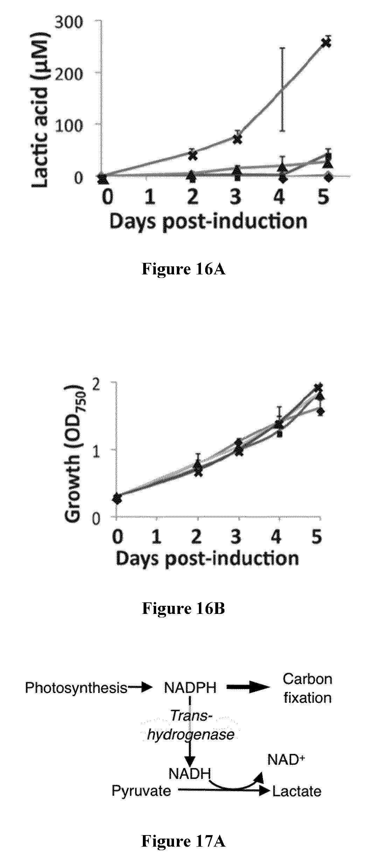

Figures 16A and 16B show lactate production and growth of *Synechococcus* strains *Synechococcus* strains expressing *E*. *coli ldhA* and *lldP* (cross), only *lldP* (triangle), only *ldhA* (square), or neither transgene (diamond). Figure 16A shows the concentration of secreted lactic acid in culture medium of these four strains. Figure 16B shows the growth of these four strains.

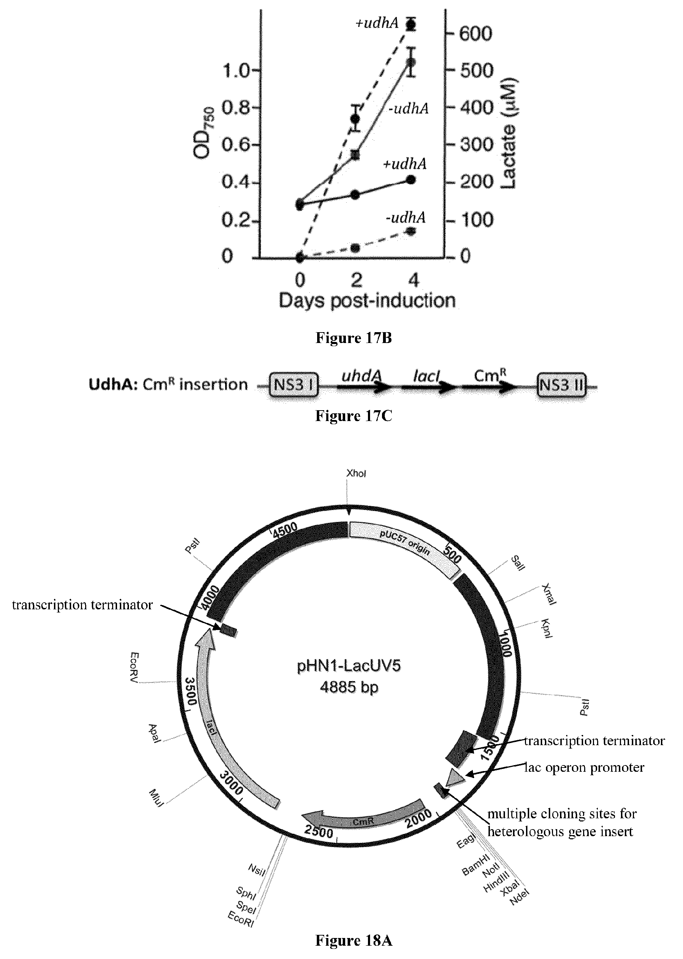

Figures 17A to 17C show rational metabolic engineering of *Synechococcus* to enhance lactate production. Figure 17A shows a schematic depiction of a model for transhydrogenase action. NADPH is the major carrier of reducing equivalents in photosynthetic microbes. Exchange with NAD⁺ is catalyzed by NADP/NAD transhydrogenase to yield NADH, the reducing agent substrate for pyruvate dehydrogenase. Figure 17B shows lactate concentrations in culture supernatants of induced *Synechococcus ldhA*+*lldP* with or without an *udhA* expression construct. Dashed lines: lactate concentrations; solid lines: bacterial density. Figure 17C shows the insertion of udhA into "neutral site" NS3 with a chloramphenicol-resistance marker.

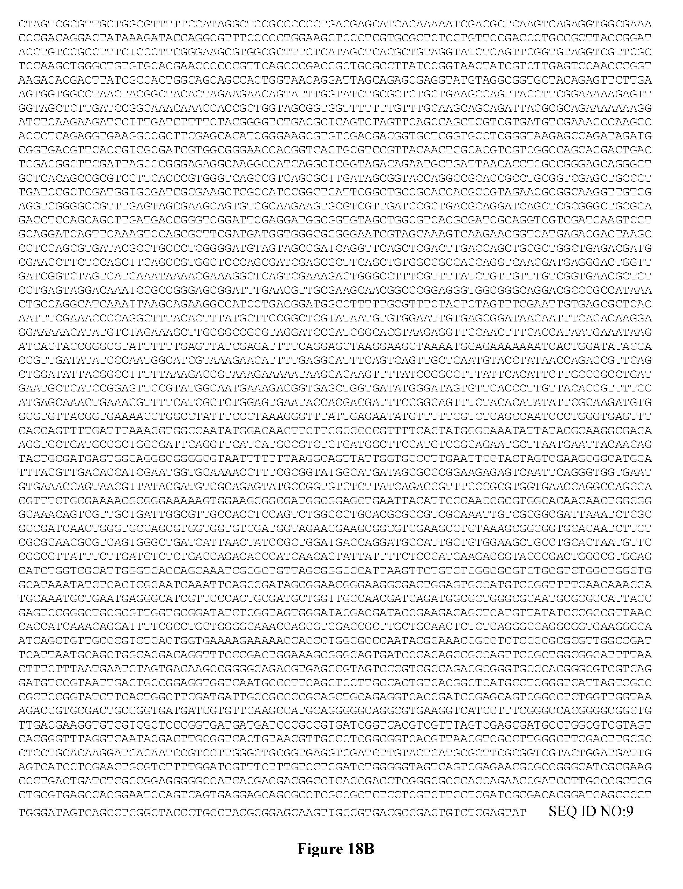

Figures 18A and 18B depict a new vector, pHN1-LacUV5, for integration of transgenes into the S. *elongatus* 7942 genome. This vector allows integration at the "NS3" site, that corresponds to a sequence within the remnant of a cryptic prophage, such that integrations are expected to have no phenotypic consequences related to integration *per se.* Figure 18A shows sites of NS3, transcription terminators, *lac* operon promoter and multiple cloning sites for genes to be expressed. The sequence of pHN1-LacUV5 vector in Figure 18B includes the origin of replication from pUC57 (bases 6 to 625); an "NS3-II" segment from bases 655 to 1554; a segment containing the *E. coli rrnB* T1 and T2 terminators from bases 1567 to 1769; the *lac*UV5 promoter from bases 1791 to 1881; a multiple cloning site including unique NdeI, XbaI, HindIII, NotI and BamHI sites from bases 1900 to 1934; a coding sequence of a chloramphenicol resistance gene from bases 2037 to 2696; an *E. coli lacI* coding sequence from bases 2839 to 3921; an *E. coli Trp* operon terminator from bases 3922 to 3972; and an "NS3-I" segment from bases 3977 to 4876. Analogous plasmids that use the weaker wild-type (not *UV5*) *lac* promoter and the stronger *Trp*(-35) *lacUV5*(-10) promoter were also constructed.

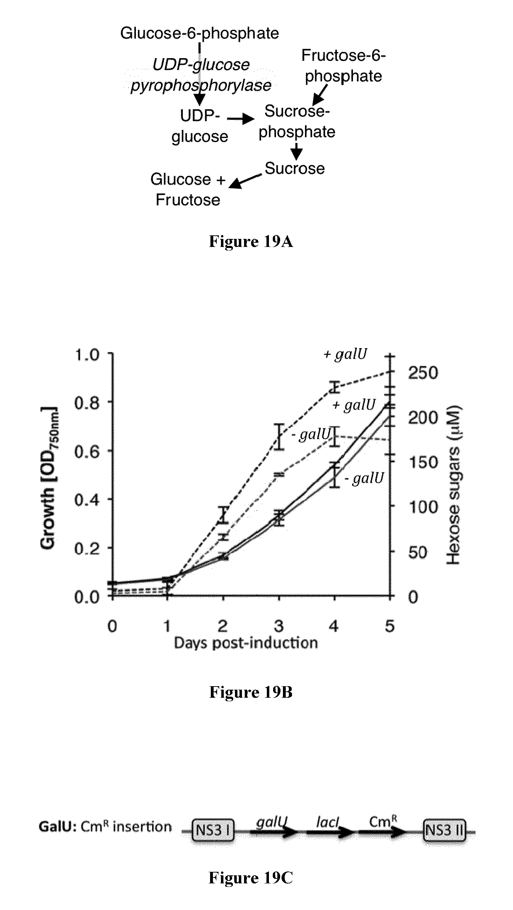

Figures 19A to 19C show the enhancement of hexose sugar production by rational metabolic engineering of *Synechococcus.* Figure 19A shows the rationale for overexpression of UDP-glucose phosphorylase. UDP-glucose and fructose-6-P are the precursors of glucose and fructose in the artificial pathway for hexose production. UDP-glucose is produced by UDP-glucose phosphorylase, encoded by the *galU* gene. Figure 19B shows the total glucose plus fructose concentrations in culture supernatants of induced *Synechococcus glf*+*invA* either with or without a *galU* expression construct. Dashed lines: glucose+fructose concentrations; solid lines: bacterial density. Figure 19C shows the insertion of *galU* into "neutral site" NS3 with a chloramphenicol-resistance marker.

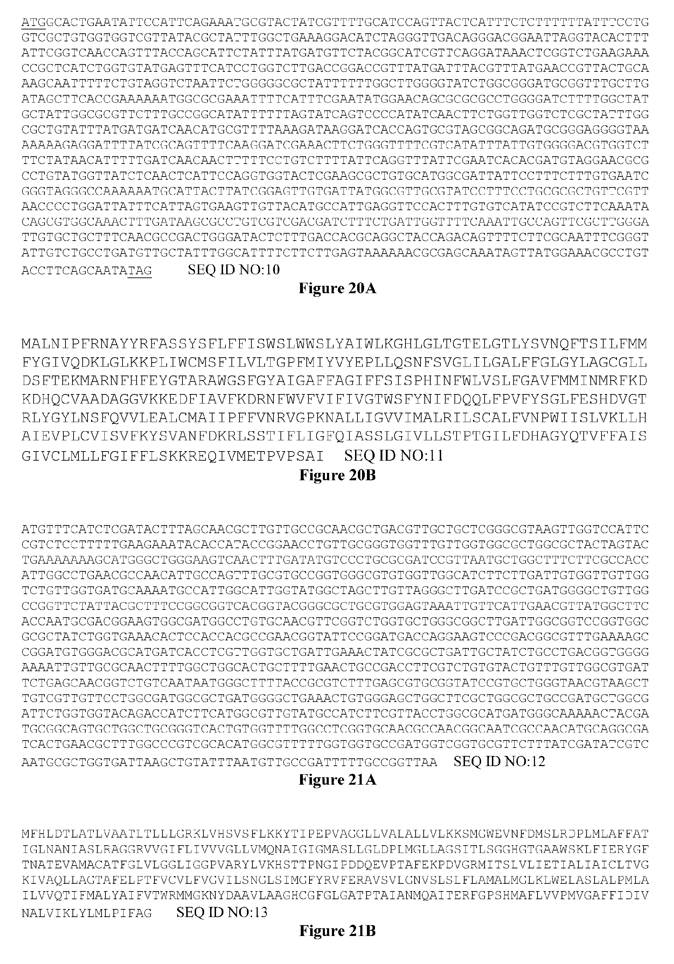

Figures 20A and 20B show the sequence for the CscB protein *of E. coli.* Figure 20A shows the nucleic acid sequence and Figure 20B shows the encoded amino acids.

Figures 21A and 21B show the sequence for the GltS protein *of E. coli.* Figure 21A shows the nucleic acid sequence and Figure 21B shows the corresponding amino acids.

### DETAILED DESCRIPTION

The present invention is not limited to the particular methodology, protocols, and reagents, etc., described herein and as such may vary. The terminology used herein is for the purpose of describing particular embodiments only, and is not intended to limit the scope of the present invention, which is defined solely by the claims.

As used herein and in the claims, the singular forms include the plural reference and vice versa unless the context clearly indicates otherwise. Other than in the operating examples, or where otherwise indicated, all numbers expressing quantities of ingredients or reaction conditions used herein should be understood as modified in all instances by the term "about." References to SEQ ID NOs and the corresponding sequence listings are part of the present specification and fully incorporated herein.

All patents and other publications identified are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

The invention provides compositions and methods for the production of useful chemicals from photosynthetic microbes. It is an object of the invention to engineer photosynthetic microbes to synthesize and secrete useful organic chemicals. Many such organic compounds are hydrophilic and require transport proteins. Many photosynthetic microbes are incapable of transporting complex organic compounds because they lack many of the transporters found in other organisms such as *E*. *coli.*

Previous efforts to metabolically engineer cyanobacteria have focused on ethanol, isobutanol, and isobutyraldehyde, which are relatively lipophilic molecules that can directly cross cell membranes. Atsumi et al., 27 Nat. Biotechnol. 1177 (2009); Deng et al., 65 Appl. Environ. Microbiol. 523 (1999). One aspect of the invention described herein relates to engineering of photosynthetic bacteria to produce a much wider variety of compounds, using transporters to export the molecule of choice.

Many photosynthetic microbes have evolved to use only CO₂, fixed or atmospheric nitrogen, and various minerals. Photosynthetic microbes are ∼1 order of magnitude more productive than conventional terrestrial plants to capture solar energy and their photosynthetic efficiencies can be >10%. Huntley et al., 12 Mitigat. Adapt. Strat. Global Change 573 (2007); Li et al., 24 Biotech. Prog. 815 (2008). This self-sufficient mode of metabolism contrasts with that of *E*. *coli,* for example, which has a wide variety of transporters. Another point of contrast is that *E*. *coli* and many other microbes secrete organic compounds as waste products. Because of the high energetic cost of fixing carbon, photosynthetic microbes generally do not secrete carbon-based waste products. The present invention provides for photosynthetic microbes that are engineered to do so, including cyanobacteria, green sulfur bacteria (including Family *Chlorobiaceae*), purple sulfur bacteria (e.g., Family *Chromatiaceae* and Family *Ectothiorhodospiraceae*) purple nonsulfur bacteria (e.g., Family *Rhodospirillaceae*), and green bacteria (including Family *Chloroflexaceae*).

The genera of cyanobacteria that may be used in various embodiments include Chamaesiphon, Chroococcus, Cyanobacterium, Cyanobium, Dactylococcopsis, Gloeobacter, Gloeocapsa, Gloeothece, Microcystis, Prochlorococcus, Prochloron, Synechococcus, Synechocystis, Chroococcidiopsis, Cyanocystis, Dermocarpella, Myxosarcina, Pleurocapsis, Stanieria, Xenococcus, Arthrospira, Borzia, Crinalium, Geitlerinema, Halospirulina, Leptolyngbya, Limnothrix, Lyngbya, Microcoleus, Oscillatoria, Planktothrix, Prochlorothrix, Pseudanabaena, Spirulina, Starria, Symploca, Trichodesmium, Tychonema, Anabaena, Anabaenopsis, Aphanizomenon, Calothrix, Cyanospira, Cylindrospermopsis, Cylindrospermum, Nodularia, Nostoc, Chlorogloeopsis, Fischerella, Geitleria, Nostochopsis, Iyengariella, Stigonema, Rivularia, Scytonema, and Tolypothri.

Cyanobacteria are photoautotrophs, able to use CO₂ as their sole carbon source and light as their energy source. Unlike certain other photosynthetic bacteria, cyanobacteria use the same photosynthetic pathway as eukaryotic cells such as algae and higher plants (the "C3" or "Calvin" cycle). Other photosynthetic bacteria use different light-harvesting pigments (bacteriochlorophyll) and metabolic pathways. Particular exemplary cyanobacteria include *Prochlorococcus, Synechococcus,* and *Synchecocystis.* A number of cyanobacterial species have been manipulated using molecular biological techniques, including the unicellular cyanobacteria *Synechocystis sp.* PCC6803 and *Synechococcus elongatus* PCC7942, whose genomes have been sequenced completely.

In one embodiment aspect, the invention provides a genetically engineered photosynthetic bacteria ("host cell") that expresses one or more heterologous enzyme and one or more heterologous transporter, wherein the transporter mediates the secretion of a product into the extracellular medium. In some embodiments, the product secreted by the recombinant bacterium is a chiral compound. Examples of chiral compounds include, without limitation, sugars and carboxylic acids, such as D-lactic acid and L-lactic acid. Exemplary sugars include lactose, galactose, glucose, fructose, a mixture of glucose and fructose, or sucrose. In another embodiment, chiral compounds include amino acids and vitamins.

Thus, in some embodiments, the transporter mediates the secretion of a product whose synthesis is enhanced by the heterologous enzyme. The heterologous enzymes can be of a type that catalyze a heterologous reaction that does not normally occur in the host organism, or can be an enzyme that is similar to an endogenous enzyme, but which catalyzes a desired reaction in a faster, more efficient, more specific, or otherwise preferable manner. For purposes of the invention, a "heterologous enzyme" is an enzyme that is not expressed naturally by the host cell. In some embodiments, heterologous enzymes include lactate dehydrogenase and invertase. For purposes of the invention, a "heterologous transporter" is a transporter that is not expressed naturally by the host cell.

In some embodiments, a gene encoding an enzyme that participates in a pathway that leads to the synthesis of a compound (such as an enzyme disclosed herein), is cloned into an expression vector for transformation into a photosynthetic bacterium. The vector includes sequences that promote expression of the transgene of interest, such as a promoter, and may include an intron sequence, a sequence having a polyadenylation signal, etc. Alternatively, if the vector does not contain a promoter in operable linkage with the gene of interest, the gene can be transformed into the cells such that it becomes operably linked to an endogenous promoter by homologous recombination or vector integration.

The heterologous genes may be "codon-optimized" for expression in an organism: the gene's nucleotide sequence has been altered with respect to the original nucleotide sequence such that one or more codons of the nucleotide sequence has been changed to a different codon that encodes the same amino acid, in which the new codon is used more frequently in genes of the host cell than the original codon. The degeneracy of the genetic code provides that all amino acids except for methionine and tryptophan are encoded by more than one codon. For example, arginine, leucine, and serine are encoded by different six different codons; glycine, alanine, valine, threonine, and proline are encoded by four different codons. Many organisms use certain codons to encode a particular amino acid more frequently than others. Without limiting any aspects of the invention to any particular mechanism, it is believed that some tRNAs for a given amino acid are more prevalent than others within a particular organism, and genes requiring a rare tRNA for translation of the encoded protein may be expressed at a low level due in part to a limiting amount of the rare tRNA. Thus, for adequate or optimal levels of expression of an encoded protein, a gene may be "codon-optimized" to change one or more codons to new codons ("preferred codons") that are among those used more frequently in the genes of the host organism (referred to as the "codon preference" of the organism). As used in the context of the invention, a "codon-optimized" gene or nucleic acid molecule of the invention need not have every codon altered to conform to the codon preference of the intended host organism, nor is it required that altered codons of a "codon-optimized" gene or nucleic acid molecule be changed to the most prevalent codon used by the organism of interest. For example, a codon-optimized gene may have one or more codons changed to codons that are used more frequently that the original codon(s), whether or not they are used most frequently in the organism to encode a particular amino acid.

A variety of gene promoters that function in cyanobacteria can be utilized in expression vectors, including (a) the *lac, tac,* and *trc* promoters that are inducible by the addition of isopropyl β-D-1-thiogalactopyranoside (IPTG); (b) promoters that are naturally associated with transposon- or bacterial chromosome-borne antibiotic resistance genes (neomycin phosphotransferase, chloramphenicol acetyltrasferase, spectinomycin adenyltransferase, etc.); (c) promoters of various heterologous bacterial and native cyanobacterial genes; (d) promoters from viruses and phages; (e) salt-inducible promoters or other types of inducible promoters; and (f) synthetic promoters.

In some instances it can be advantageous to express a heterologous enzyme at a certain point during the growth of the transgenic host to minimize any deleterious effects on the growth of the transgenic organism and/or to maximize yield of the desired product. In these instances one or more exogenous genes introduced into the transgenic organism can be operably linked to an inducible promoter. The promoter can be a *lac* promoter, a *tet* promoter (e.g., U.S. Patent No. 5,851,796), a hybrid promoter that includes either or both of portions of a *tet* or *lac* promoter, a hormone-responsive promoter (e.g., an ecdysone-responsive promoter, e.g., U.S. Patent No. 6,379,945) a metallothionine promoter (U.S. Patent No. 6,410,828), or a promoter that can be responsive to a chemical such as, for example, salicylic acid, ethylene, thiamine, or BTH (U.S. Patent No. 5,689,044). An inducible promoter can also be responsive to light or dark (U.S. Patents No. 5,750,385; No. 5,639,952) or temperature (U.S. Patent No. 5,447,858; Abe et al., 49 Plant Cell Physiol. 625 (2008); Shroda et al., 21 Plant J. 121 (2000)), or copper level. Surzycki et al., 104 PNAS 17548 (2007). The promoter sequences can be from any organism, provided that they are functional in the host organism. Inducible promoters as used in the constructs of the present invention can use one or more portions or one or more domains of the aforementioned promoters or other inducible promoters fused to at least a portion of a different promoter that operates in the host organism to confer inducibility on a promoter that operates in the host species. Promoters isolated from cyanobacteria that have been used successfully include *secA* (controlled by the redox state of the cell); *rbc* (Rubisco operon); *psaAB* (light regulated); *psbA* (light-inducible); and *nirA* (NH₃NO₃ regulated).

Likewise, a wide variety of transcriptional terminators can be used for expression vector construction. Examples of possible terminators include, but are not limited to, psbA, psaAB, rbc, secA and T7 coat protein, as are known in the art.

In some embodiments, a gene encoding an enzyme that participates in a pathway that leads to the synthesis of the chiral compound (such as an enzyme disclosed herein), is cloned into an expression vector for transformation into a photosynthetic bacterium. The vector includes sequences that promote expression of the transgene of interest, such as a promoter, an intron sequence, a sequence having a polyadenylation signal, etc. Alternatively, if the vector does not contain a promoter in operable linkage with the gene of interest, the gene can be transformed into the cells such that it becomes operably linked to an endogenous promoter by homologous recombination or vector integration.

Transformation vectors can also include a selectable marker, such as but not limited to a drug resistance gene, an herbicide resistance gene, a metabolic enzyme or factor required for survival of the host (for example, an auxotrophic marker), etc. Transformed cells can be optionally selected based upon the ability to grow in the presence of the antibiotic or other selectable marker under conditions in which cells lacking the resistance cassette or auxotrophic marker would not grow. In some embodiments a non-selectable marker may be present on a vector, such as a gene encoding a fluorescent protein or enzyme that generates a detectable reaction product. In an alternative transformation strategy, selectable or non-selectable markers can be provided on a separate construct, where both the gene-of interest construct and the selectable marker construct are used together in transformation protocols, and selected transformants are analyzed for co-transformation of the construct that includes the gene-of-interest. *See, e.g.,* Kindle, 87 PNAS 1228 (1990); Jakobiak et al., 155 Protist 381 (2004).

In some embodiments, a vector is designed for integration of the heterologous nucleic acid sequence into the host genome. Example vectors can be (a) targeted for integration into a bacterial chromosome by including flanking sequences that enable homologous recombination into the chromosome; (b) targeted for integration into endogenous host plasmids by including flanking sequences that enable homologous recombination into the endogenous plasmids; and/or (c) designed such that the expression vectors replicate within the chosen host. Artificial chromosome vectors can also be used for the transformation of photosynthetic microorganisms when more than one gene that encodes an enzyme that participates in the synthesis and/or a transporter that enables secretion of a product as described herein is transformed into an organism.

In some embodiments, the transporter is a member of the major facilitator superfamily. This family of proteins includes the Glut family of glucose transport proteins in humans and other mammals, the HXT family of glucose transporters in yeast, the *lacY* gene product, the *lldP* lactate transporter of *E*. *coli* or another bacterium that performs anaerobic metabolism, a mammalian citric acid transport protein, an ion-coupled sucrose transporter such as *cscB,* and the glpT glycerol phosphate transporter. The *GLF* protein of *Z*. *mobilis* is related to the glucose transporters from yeast and humans. These proteins have twelve membrane-spanning α-helices that form two rigid domains of six helices each. The subset of these proteins that includes the Glut, HXT and *GLF* proteins generally mediate transport of glucose, but their ability to transport other sugars such as fructose varies depending on the specific protein.

In one embodiment, photosynthetic bacteria are engineered to synthesize a mixture of glucose and fructose. This is achieved by the expression of a heterologous invertase enzyme that catalyzes the breakdown of sucrose into glucose plus fructose. Synthesis of sucrose within the cell can be induced in certain cyanobacteria such as S. *elongatus* 7942 and *Synechocystis sp.* 6803 by addition of salt to the medium; the sucrose is thought to maintain osmotic balance. Blumwald et al., 80 PNAS 2599 (1983). In alternative embodiments, the mechanism for producing sucrose utilizes key enzymes such as sucrose phosphate synthase and sucrose phosphate phosphatase expressed under the control of an inducible promoter with a different mode of regulation. In further embodiments, a heterologous hexose transporter is also expressed in the cyanobacteria, and this protein mediates the transport of glucose and fructose out of the cell.

When such a protein is co-expressed in cyanobacteria with invertase and sucrose synthesis is induced, the hexose products from the invertase reaction are secreted from the cell. For example, when the *GLF* and intracellular invertase proteins of *Z*. *mobilis* are expressed in *S. elongatus* 7942, intracellular sucrose is cleaved to glucose and fructose and these products are secreted into the medium.

In further embodiments, genetically engineered photosynthetic bacteria described herein for production of hexose sugars may be further optimized as follows. For example, it is sometimes useful to introduce a reduction-of function mutation, such as a deletion, in an endogenous hexokinase gene, an endogenous glucokinase gene, or both. Without wishing to be bound by theory, this has the effect of reducing metabolism of glucose and/or fructose that is the desired product. In alternative embodiments, an additional gene that enhances production of UDP-glucose, which is a precursor of sucrose, can be further expressed in engineered photosynthetic microbes. For example, the *galU* gene that encodes a UDP-glucose pyrophosphorylase of a bacterium such as *E*. *coli* or a cyanobacterium can be used. Additional embodiments include over-expression of the genes that produce sucrose, which are sucrose phosphate synthase and sucrose phosphate phosphatase.

Another type of optimization relates to the type of promoter that is used for expression of the heterologous enzyme and/or transporter. In some embodiments, the enzyme is expressed from an inducible promoter. In another embodiment, the promoters used are selected from the lac operon promoter, a nitrogen-sensitive promoter, and a salt-inducible promoter. For example, the promoters for cyanobacterial nitrate reductase or nitrite reductase genes are repressed by ammonia and induced by nitrate; these are used as nitrogen-sensitive promoters.

It is convenient to use an inexpensive inducing agent, such as NaCl, rather than a more costly compound such as IPTG. Thus, NaCl-inducible promoters can be used for expression of the heterologous enzyme and/or transporter. The NaCl-inducible promoters for the cyanobacterial sucrose phosphate synthase and sucrose phosphate phosphatase genes can be used for this purpose.

In another embodiment, lactic acid is produced using the methods and organisms of the invention. In one embodiment, lactate dehydrogenase and a lactate transporter are expressed in a cyanobacterium. The lactate dehydrogenase (*ldhA*) can be derived from any organism, such as from Gram-negative organism such as *E*. *coli,* from another cyanobacterium, or from a mammal such as a human. For example, lactate dehydrogenase of *E*. *coli* or any of a wide variety of other organisms is expressed in a cyanobacterium, and a lactate/H⁺ co-transporter is also expressed. The *E*. *coli* lactate/H⁺ transporter illustrates many of the properties of this protein family. The *E*. *coli* protein is encoded by the *lldP* gene and is able to transport L-lactate, D-lactate, and glycolate, making it useful for transporting a variety of molecules from engineered organisms.

This lactate transporter protein, like the hexose transporter described above, is also in the major facilitator superfamily, of which members have twelve membrane-spanning α-helices, are thought to form two rigid domains of six helices each, and generally lack cleaved signal sequences. In the context *of E. coli* growing anaerobically, lactate is normally secreted as a waste product. The co-transport of a proton adds to the electrochemical gradient and export of lactate is thus an energy-generating step. When cyanobacteria are cultured, the pH of the medium often rises to pH 10 or pH 11 (Becking et al., 68 J. Geol. 243 (1960)). Thus, co-transport of lactate and a proton is strongly driven by the pH gradient in such cultures, and the reverse transport is negligible.

Another aspect of the invention provides for recombinant photosynthetic bacteria, such as cyanobacteria, that express a heterologous transporter that is coupled to the pH gradient across a cell membrane. In some embodiments, genetically engineered photosynthetic bacteria, such as cyanobacteria, can express a heterologous transporter that mediates the symport or antiport of a product and an inorganic ion. Examples of an inorganic ion include, but not limited to, a proton, a sodium ion, a potassium ion, a chloride ion, and a hydroxide ion. In additional embodiments, genetically engineered photosynthetic bacteria, such as cyanobacteria, can express a heterologous transporter that co-transports a proton with a metabolite. In further embodiments, the invention provides genetically engineered photosynthetic bacteria, such as cyanobacteria, that express a heterologous transporter that co-transports a sodium ion with a metabolite.

According to the invention, the principle of coupling transport to the pH gradient is generalized. Other bacteria express a number of transporters that, like the *lldP* protein, co-transport a given molecule with a proton or with a cation such as sodium that is coupled to the proton gradient. Such other bacteria generally secrete protons into the extracellular space, resulting in acidification of the medium. The secreted protons act as a form of stored energy, which is used when nutrient molecules are co-transported with a proton or sodium ion into the cell. Most cyanobacteria tend to alkalinize their growth medium, which differentiates them from other bacteria. Accordingly, such alkalinization will cause transporters that have evolved for nutrient import or energy generation to promote export of nutrients when the transporter is artificially expressed in a cyanobacterium. In some embodiments, the transporter can be a lactic acid/proton symporter, a sucrose proton symporter, a lactose/proton symporter, a dicarboxylic acid/proton symporter, an amino acid/proton symporter, a citrate/proton symporter, an amino acid/sodium ion symporter, or an amino acid/hydroxide ion antiporter. Examples of additional proton-coupled transporters include the sucrose transporter CscB (Vadyvaloo et al., 358 J. Mol. Bio. 1051 (2006)), the lactose permease *of E. coli* encoded by the *lacY* gene (Guan et al., 35 Ann. Rev. Biophys. Biomol. Str. 67 (2006)), and the mammalian H⁺/peptide transporter PepT1H (Chen et al., 272 Biochem. Biophy. Res. Commn. 726 (2000)).

Because cyanobacteria start with light and CO₂ as feedstocks, the cost of production of any carbon-based molecule is a function of the number of photons needed to drive the synthesis of the molecule and the efficiency of the engineered pathway. For example, the cost of lactic acid is currently higher than the cost of sugar because sugar is used as a feedstock to produce lactic acid. Without wishing to be bound by theory, production of lactic acid by cyanobacteria can abolish this distinction, as the rates of production of sugars and lactic acid can become comparable.

Another aspect of the invention provides for the expression of a soluble NAD(P)H transhydrogenase in photosynthetic microbes engineered to express lactate dehydrogenase or other enzymes that perform redox reactions. In addition embodiments, it is often useful to eliminate, for example by mutation, any membrane-associated NAD(P) transhydrogenase. Without wishing to be bound by theory, one rationale is that most lactate dehydrogenase enzymes use NADH, rather than NADPH, as a substrate. During photosynthesis, NADPH is directly produced from electrons that emerge from Photosystem I via ferredoxin and ferredoxin-NADP reductase. Thus, in photosynthetically active cells, levels of NADPH generally exceed levels of NADH. Expression of a soluble NAD(P)H transhydrogenase in sufficient quantities thus moves the balance of reducing equivalents between NAD and NADP toward equilibrium.

The lactate and glucose transporters with twelve membrane-spanning regions represent a large class of such transporters, termed the major facilitatory superfamily. Marger et al., 18 Trends Biochem. Sci. 13 (1993). The lactate and glucose transporters, and many other proteins in this family, do not have signal sequences and the mechanism by which the proteins insert into the membrane is unknown. The results obtained here indicate that members of this superfamily can be co-expressed with appropriate enzymes, providing export of the desired product.

As noted herein, a wide variety to photosynthetic bacteria can be manipulated using the methods of the present invention. For example, a heterologous over-expression system based on *Rhodospirillum rubrum* has been reported whereby proteins can be expressed under control of the regulatable promoters of the *puh* and *puf* operons.

The photosynthetic apparatus in *Rhodospirillum* is less evolved than *Rhodobacter*, in which a full length cDNA encoding yellow tail (*Seriola quinqueradiata*) growth hormone was cloned into an expression vector (under the *lac* promoter of *E. coli*) that contained a *Rhodobacter*-specific replicon. The resulting plasmid was introduced by transformation into the marine purple non-sulfur photosynthetic bacterium *Rhodobacter sp.* strain NKPB0021. The plasmid was maintained as an autonomous replicon and showed good stability in the absence of antibiotics. Burgess et al., 15 Biotech. Letts. 111 (1993).

Further regarding *Rhodobacter*, a photosynthetic variant was engineered to produce hydrogen in a light-independent manner by adding pyruvate lyase and formate lyase complex to a photosynthetic strain of *Rhodobacter sphaeroides* using *E*. *coli* conjugation. U.S. Patent Appl. Pub. No. 2010/0003734. A versatile *Rhodobacter* heterologous protein expression vector comprising a promoter nucleic acid sequence operable in a *Rhodobacter*, a nucleic acid sequence encoding an extended purification tag, a cloning cassette comprising a multiple cloning site and a selection marker has been described (WO 07/038746) and may be adapted for use in the present methods.

Another cloning system has been used to produce branched-chain alcohols via heterologous alcohol dehydrogenase in *Synechococcus elongatus.* More specifically, a construct combining *Saccharomyces cerevisiae* pyruvate decarboxylase gene and *S*. *cerevisiae* alcohol dehydrogenase gene cloned into *S. elongatus* PCC 7942. Also, a *Lactococcus lactis KDCa* gene was combined with *S. cerevisiae ADH2* gene and transformed into *S. elongatus* cells as described by Golden and Sherman (158 J. Bacteriol. 36 (1984)). Additionally, a *Lactococcus lactis KDCa* gene was combined with S. *cerevisiae ADH2* gene and transformed into *S. elongatus* cells as described by Golden and Sherman (1984). See U.S. Patent Appl. Pub. No. 2010/0151545.

Another cyanobacterium, *Synechocystis* PCC 6803, was transformed with plasmids encoding codon-modified *S. cerevisiae PDC1* and *ADH2* genes, as described by Zang et al. (45 J. Microbio. 241 (2007)); and the acetolactate synthase gene from *Synechocystis sp.* PCC 6803 was redesigned and cloned into wild-type *Synechocystis* as described by Zang et al., 2007. See U.S. Patent Appl. Pub. No. 2010/0151545.

Others have described introducing O₂-tolerant hydrogenase from purple sulfur photosynthetic *Thiocapsa roseopersicina* into cyanobacteria *Synechococcus* and *Synechocystis* using a commercial *E. coli* shuttle vector; and introducing O₂-tolerant hydrogenase from purple non-sulfur photosynthetic Rubrivivax gelatinosus CBS into cyanobacteria Synechococcus and Synechocystis. J. Craig Venter Institute, Rockville, MD.

Further regarding the cyanobacterium *Synechococcus*, the eicosapentaenoic acid (EPA) synthesis gene cluster from an EPA-producing bacterium, *Shewanella sp.* SCRC-2738, was cloned into a broad-host range vector and then introduced into the marine *Synechococcus sp.* NKBG15041 c, by conjugation. The transgene was expressed and EPA produced in a variety of light-levels and temperatures. Yu et al., 35 Lipids 1061 (2000). Similarly, recombinant *Synechococcus sp.* PCC 7942 was constructed for the photosynthetic conversion of CO₂ to ethylene by inserting the ethylene-forming enzyme of *Pseudomonas syringae.* Sakai et al., 84 J. Ferment. Bioeng. 434 (1997). *Synechococcus sp.* PCC 7942 was also transformed with a recombinant plasmid harboring poly-(hydroxybutyrate) (PHB)-synthesizing genes from the bacterium *Alcaligenes eutrophus.* The transformant accumulated PHB in nitrogen-starved conditions, the PHB content held stable during a series of batch cultures, and the yield was increased by CO₂-enrichment. Suzuki et al., 18 Biotech. Lett. 1047 (1996); Takahashi et al., 20 Biotech. Lett. 183 (1998).

Another effort showed that the *E*. *coli lacZ* gene could be expressed in *Synechococcus* R2 PCC7942 both as a plasmid-borne form and integrated into the chromosome. A promoterless form of the *lacZ* gene was used as a reporter gene to make transcriptional fusions with cyanobacterial promoters using a shuttle vector system and also via a process of integration by homologous recombination. *Synechococcus* R2 promoter-*lacZ* gene fusions were used to identify CO₂-regulated promoters by quantitatively assessing the β-galactosidase activity under high and low CO₂ conditions, which detected several promoters induced under low CO₂ conditions. Scanlan et al., 90 Gene 43 (1990).

Several vectors have been described for the expression of recombinant genes in the cyanobacterium, *Anacystis,* including recombinant plasmids capable of integration into the chromosome of *A. nidulans R2* (Elanskaya et al., 11 Molec. Genet. Microbio. Virol. 20 (1985)); a hybrid plasmid capable of transforming *E. coli* and *A. nidulans* (Friedberg & Seijffers, 22 Gene 267 (1983)); and specifically *E. coli* K12 and *A. nidulans* R2 (Kuhlemeier et al., 184 Mol. Gen. Genet. 249 (1981).

Regarding the nitrogen fixing cyanobacterium *Anabaena sp.* PCC7120, a recombinant strain was designed to constitutively express the Ca²⁺-binding photoprotein apoaequorin in a study of Ca²⁺ transients in response to heat and cold shock signaling. Torrecilla et al., 123 Plant Physiol. 161 (2000). Hence, the methods of the present invention can be applied to a wide variety of photosynthetic bacteria.

In some embodiments, photosynthetic bacteria expressing a heterologous enzyme and a heterologous transporter as described herein can be further engineered to express one or more genes for enhancing production of hydrophilic products. In one embodiment, the additional genes encode enzymes for enhancing synthesis of intracellular precursors. For example, the *galU* gene from a bacterium such as *E. coli* can be further expressed in engineered photosynthetic microbes to increase intracellular sucrose, which is the precursor for production of glucose and fructose. Alternatively, the *udhA* gene from a bacterium such as *E*. *coli* can be further introduced into engineered photosynthetic microbes to increase intracellular NADH, which is the reducing substrate for lactate dehydrogenase required for conversion of pyruvate to lactate. Alternative embodiments include reduction-of function mutation, such as deletion of genes that causes a decrease in intracellular precursors or synthesized hydrophilic products via different cellular processes such as metabolism, degradation, post-modification, or co-binding with other products.

Still another aspect of the invention provides for a method for producing a hydrophilic product, the method comprising culturing a microbe according to the first aspect of the invention in the presence of light, CO₂, appropriate minerals and water, and obtaining said the hydrophilic product from a supernatant of the cultured microbe. In some aspects the method further includes providing an inducing agent to induce the expression of at least one heterologous gene expressed in photosynthetic microbe. In one embodiment, the heterologous gene encodes an enzyme or a transporter. In further embodiments, the invention presented herein contemplates an attractive and economically feasible strategy for biofuel production using cyanobacteria in photobioreactors.

In another aspect, the invention provides a method for producing a hydrophilic product, the method comprising culturing a microbe according to the second aspect of the invention in the presence of light and CO₂, appropriate minerals and water, and obtaining said the hydrophilic product from a supernatant of the cultured microbe. In some aspects the method further includes providing an inducing agent to induce the expression of at least one heterologous gene expressed in photosynthetic microbe. In one embodiment, the heterologous gene encodes an enzyme, a transporter or both.

In various embodiments, products can be purified from culture supernatants by standard techniques known to a skilled artisan. *See, e.g.,* Ikeda, in 79 ADVANCES BIOCHEMICAL ENGIN./BIOTECH.: MICROBIAL PROD. OF AMINO ACIDS, 1-35 (Faurie & Thommel, eds., Springer-Verlag, Berlin Heidelberg, Germany (2003)). Sugars are purified by concentration and crystallization or simply obtained as sugar solutions with minor inorganic contaminants from the medium after filtration to remove bacteria. Organic acids such as succinate or citrate are purified by acidification of the culture supernatant, followed by precipitation/crystallization of the organic acid, according to standard procedures well-known in the art.

*In some embodiments of the present invention may be defined in any of the following numbered paragraphs:*

A recombinant photosynthetic bacterium comprising at least one heterologous enzyme and at least one heterologous transporter, wherein said transporter mediates the secretion of a product whose synthesis is enhanced by said enzyme.

The recombinant photosynthetic bacterium of paragraph [0084], wherein the transporter is a member of the major facilitator superfamily.

The recombinant photosynthetic bacterium of paragraph [0084], wherein the heterologous transporter encoded by a gene selected from the group consisting of: the Glut family of glucose transport proteins in humans and other mammals; the HXT family of glucose transporters in yeast; *lacY; lldP* lactate transporter of *E*. *coli* or another bacterium that performs anaerobic metabolism; a mammalian citric acid transport protein; the *glpT* glycerol phosphate transporter; and *GLF* of *Z. mobilis.*

The recombinant photosynthetic bacterium of paragraph [0084], wherein the enzyme is an invertase.

The recombinant photosynthetic bacterium of paragraph [0086] or [0087], further comprising a reduction-of-function mutation in an endogenous hexokinase gene, an endogenous glucokinase gene, or both.

The recombinant photosynthetic bacterium of any of paragraphs [0084] to [0088], wherein the product is a chiral compound.

The recombinant photosynthetic bacterium of paragraph [0089], wherein the chiral compound is a hexose sugar.

The recombinant photosynthetic bacterium of paragraph [0090], wherein the hexose sugar is glucose, fructose, or a mixture of glucose and fructose.

The recombinant photosynthetic bacterium of paragraph [0084], wherein the enzyme is lactate dehydrogenase and the transporter is a lactate transporter.

The recombinant photosynthetic bacterium of paragraph [0092], wherein the lactate transporter is encoded by the *lldP* gene *of E. coli.*

The recombinant photosynthetic bacterium of paragraph [0092] or [0093], further comprising a reduction-of-function mutation in malate dehydrogenase, pyruvate dehydrogenase complex, phosphoenolpyruvate carboxylase, phosphoenolpyruvate synthase.

The recombinant photosynthetic bacterium of any of paragraphs [0092] to [0094], wherein the product is lactic acid.

The recombinant photosynthetic bacterium of any of paragraphs [0084] to [0095], wherein the enzyme is expressed from an inducible promoter.

The recombinant photosynthetic bacterium of paragraph [0096], wherein the photosynthetic bacterium is a cyanobacterium.

The recombinant photosynthetic bacterium of paragraph [0097], wherein the cyanobacterium is selected from *Prochlorococcus spp., Synechococcus spp.,* and *Synchecocystis spp.*

The recombinant photosynthetic bacterium of any one of paragraphs [0084] to [0098], wherein the photosynthetic bacterium is additionally engineered to express a soluble NAD(P) transhydrogenase.

The recombinant photosynthetic bacterium of any of paragraphs [0084] to [0098], wherein the photosynthetic bacterium is additionally engineered to express a UDP-glucose phosphorylase.

A recombinant photosynthetic bacterium comprising at least one heterologous transporter, wherein said transporter mediates the symport or antiport of a product and an inorganic ion.

The recombinant photosynthetic bacterium of paragraph [00101], wherein said inorganic ion is selected from the group consisting of a proton, a sodium ion, a potassium ion, a chloride ion, and a hydroxide ion.

The recombinant photosynthetic bacterium of paragraph [00101], wherein said transporter is selected from the group consisting of a lactic acid/proton symporter, a sucrose proton symporter, a lactose/proton symporter, a dicarboxylic acid/proton symporter, an amino acid/proton symporter, a citrate/proton symporter, an amino acid/sodium ion symporter, and an amino acid/hydroxide ion antiporter.

The recombinant photosynthetic bacterium of paragraph [00101], wherein the heterologous transporter encoded by a gene selected from the group consisting of: the sucrose transporter CscB; the lactose permease of E. coli encoded by the lacY gene; the mammalian H+/peptide transporter PepT1H; the glutamate transporter GltS and the lactate transporter *lldP.*

The recombinant photosynthetic bacterium of paragraph [00101], wherein the product is a chiral product.

The recombinant photosynthetic bacterium of paragraph [00101], wherein the product is an amino acid.

The recombinant photosynthetic bacterium of paragraph [00106], wherein the amino acid is glutamate.

The recombinant photosynthetic bacterium of paragraph [00101], wherein the product is a sugar.

The recombinant photosynthetic bacterium of paragraph [00101], further comprising at least one more heterologous gene.

The photosynthetic bacterium of paragraph [00109], wherein the heterologous gene encodes an enzyme or a transporter.

The photosynthetic bacterium of paragraph [00110], wherein the enzyme is *ldhA.*

A method for producing a product, said method comprising culturing a recombinant photosynthetic bacterium of any of paragraphs [0084] to [00100] in the presence of light and carbon dioxide, and obtaining the product from the supernatant of the cultured bacterium.

A method for producing a product, said method comprising culturing a recombinant photosynthetic bacterium of any of paragraphs [00101] to [00111] in the presence of light and carbon dioxide, and obtaining the product from the supernatant of the cultured bacterium.

### EXAMPLES

The following Examples are intended to further illustrate certain embodiments of the invention and are not to be construed as limiting the scope of the invention.

The sequences of the integrated transgenes used in the Examples have been deposited in the GenBank database under accession numbers HM026754 *(ldhA* in NS2), HM026755 *(lldP* in NS1), HM026756 *(glf in* NS1), HM026757 *(invA* in NS2), and HM026758 (novel transgene in NS3).

### Example 1. Engineering of a photosynthetic bacterium for production of sugars

To engineer a photosynthetic microbe to produce the hydrophilic compounds, e.g., glucose and fructose or lactic acid, enzymes for intracellular synthesis of these hydrophilic molecules of interest and also relevant transporters for export of the synthesized hydrophilic compounds can be expressed in the photosynthetic microbe. In one embodiment, an engineered photosynthetic microbe that produces and secretes intracellular sugars, particularly glucose and fructose, was constructed as follows. The specific microbe used in this Example was the cyanobacterium *S. elongatus* PCC7942 *(Synechococcus),* although a wide variety of cyanobacteria and other microbes could be used. *S. elongatus* 7942 is a fresh water cyanobacterium whose growth is somewhat inhibited by salt. In response to salt water, e.g., NaCl treatment, *S. elongatus* 7942 synthesizes sucrose, which remains intracellular and serves to osmotically balance the inside of the cell with the extracellular medium. Blumwald et al., 80 PNAS 2599 (1983); Miao et al., 218 FEMS Microbio. Lett. 71 (2003).

*Engineering strategy:* To engineer recombinant *S. elongatus* 7942 that produce and secrete a mixture of glucose and fructose, two genes were introduced into S. *elongatus* 7942: (a) the *Zymomonas mobilis invA* gene encoding a soluble, cytoplasmic invertase (Yanase et al., 55 Agric. Biol. Chem. 1383 (1991)) to cleave NaCl-induced sucrose, and (b) the Z. *mobilis glf* gene encoding a glucrose- and fructose-facilitated diffusion transporter, which allows export of the sugars from the cell. Barnell et al., 172 J. Bacteriol. 7227 (1990). The GLF protein belongs to the major facilitator superfamily whose members have twelve transmembrane α-helical segments and generally lack cleaved signal sequences. DiMarco et al., 49 Appl. Environ. 151 (1985); Snoep et al., 176 J. Bacteriol. 2133 (1994). The GLF protein facilitates diffusion of glucose and fructose in either direction across the membrane, and the transported sugar is not phosphorylated. Marger et al., 18 Trends Biochem. Sci. 13 (1993). According to an aspect of the invention, when S. *elongatus* 7942 is placed in a high-salt environment and the *invA* and glf genes are expressed, the bacterium produces intracellular sucrose, which is then cleaved into glucose and fructose by the invertase protein and allowed to be transported across the cell membrane into the medium by the hexose transporter encoded by *glf.* This general concept is illustrated in Figure 1.

*Plasmid and strain construction:* The heterologous *invA* and *glf* genes were inserted into plasmid vectors that mediate integration into the S. *elongatus* 7942 genome by homologous recombination using neutral sites (Clerico et al., 362 Methods Mol. Biol. 155 (2007)) that can tolerate insertion with no phenotypic effects (Clerico et al., 362 Mets. Mol. Bio. 155 (2007); Mackey et al., 362 Mets. Mol. Bio. 15 (2007)). Neutral site 1 (NS1) and NS2 are present in plasmids DS1321 and DS21, which confer spectinomycin and kanamycin resistance, respectively, and contain E. *coli lacI* and an isopropyl-β-D-thiogalactopyranoside (IPTG)-regulated *trp-lac* strong promoter. Diagrams of these vectors are shown in Figure 2.

The *invA* and glf genes from Z. *mobilis* were obtained from Genscript (Piscataway, NJ). The two genes were codon-optimized for expression in *Synechococcus* and were synthesized so that they contained a C-terminal His₆ tag. These genes were inserted into DS21 and DS1321 by using standard procedures. The *invA* gene was inserted in NS 1 along with a spectinomycin-resistance marker and a *lacI* gene; the *invA* gene was expressed from an *E. coli trp*/*lac* promoter and also encoded a C-terminal His₆ tag as an epitope for detection in western blots. The *glf* gene was inserted in NS 2 along with a kanamycin-resistance marker and a *lacI* gene; the *glf* gene was expressed from an *E. coli lac* promoter and also encoded a C-terminal His₆ tag as an epitope for detection in western blots. The sequences of these regions are shown in Figures 3 and 4, respectively. The E. *coli lacI* gene was also expressed in *Synechococcus* from its own promoter. Transformation of *Synechococcus* was performed as described previously. Clerico et al., 362 Mets. Mol. Bio. 155 (2007). Integration of vectors into neutral sites was verified by PCR to demonstrate the presence of appropriate novel chromosome-transgene junctions and the absence of uninserted sites.

*Activity of transgenes in vivo:* Accordingly, strains of *S. elongatus* 7942 were constructed that express *invA* and *glf, invA* alone, *glf* alone, or neither transgene. To control for the presence of antibiotic resistance genes and lacI genes and to allow use of the same antibiotics in cultures being compared, 'empty' insertions containing only these genes were made in some strains. The expression and functions of heterologous genes were determined by performing direct assays, as well as by examining secretion of molecules of interest in engineered *Synechococcus* strains. The functionality of the cloned *Z*. *mobilis invA* and glf genes in E. *coli* was shown as follows: the glf expression plasmid rescued growth of an E. *coli ptsI* mutant (CHE30) (Plumbridge, 33 Mol. Microbio. 260 (1999)) on glucose, and *E. coli* DH5α containing the *invA* expression plasmid was able to use sucrose as a carbon source. Expression of *InvA*-HiS₆ in *Synechococcus* was observed by western blotting and was IPTG-inducible, although *GLF*-His₆ were not detected (Figure 5A), perhaps due to low levels of expression combined with poor extraction from membranes or His₆ tag being proteolytically removed. The invertase activity in *Synechococcus* extracts was increased ∼18-fold by expression of *invA* (Figure 5C). The functionality *of glf* in *Synechococcus* was shown by adding 500 µM glucose or fructose to the culture medium, which allowed slow, heterotrophic growth of the cyanobacteria in the dark, while the sugar disappeared from the medium (Figure 5D).

*Experimental method for invertase activity assay:* Crude cell extracts of *Synechococcus* strains expressing *invA* and glf were prepared from a culture that had been induced with 100 µM IPTG for 3 days. Cell pellets were resuspended in invertase assay buffer and disrupted by sonication on ice for 2.5 sec followed by a 5-sec break, using a total sonication time of 5 min. Cell debris was removed by centrifugation. Invertase activity was measured as described (Yanase et al., 55 Agric. Biol. Chem. 1383 (1991)), using an assay mixture containing crude cell extracts in 100 mM sodium acetate buffer (pH 5) at 30°C. The reaction was started by addition of 150 mM sucrose. After 10 min incubation, the reaction was stopped by incubation at 100°C for 2 min. The heat-denatured proteins were pelleted, and the glucose concentration in the supernatant was determined using a sucrose-glucose-fructose assay kit (Megazyme Intl., Wicklow, Ireland). To determine the invertase specific activity in the crude cell extracts, the protein concentration was measured by performing a Bradford protein assay (Bio-Rad).

*Secretion of sugars from engineered cynaobacteria:* Coexpression of catabolic enzymes and transporters led to secretion of hexose sugars. Typical results are shown in Figures 6, 7 and 8. These results are from a representative experiment in which on Day 0, engineered *Synechococcus* containing the *invA* and *glf* transgenes was treated with 200 mM NaCl to induce sucrose synthesis and 100 micromolar IPTG to induce expression of the *invA* and *glf* genes. Cells were grown in alternating 18 hours of light, 6 hours of dark, in atmospheric levels of CO₂ at 30°C. The strains tested were a control strain carrying "empty vector" chromosomal insertions, strains carrying the *glf* gene or the *invA* gene with empty insertions corresponding to the other gene, and a strain carrying both the *glf and invA* genes. These strains are schematically shown in Figure 2.

Figure 6 shows that the *Synechococcus* strain expressing the *invA* and *glf* genes produced much larger amounts of glucose and fructose than the other strains, and the maximum total concentration was about 200 µM when both *invA* and *glf* genes were expressed (Figures 6A-6B). Thus, the combination of both the *invA* and *glf* genes is particularly useful for production of glucose and fructose by photosynthetic bacteria.

The glucose levels in the medium were significantly lower than the fructose levels, and the fructose levels declined after reaching a maximum level as the cell density increased (Figures 6A and 6B); these observations suggest that the engineered cyanobacteria could re-transport glucose and fructose back into the cell and metabolize them. This idea is further supported by observations below. According to the invention, the yield of glucose and fructose is further improved by the introduction of mutations such as reduction-of-function mutations in hexokinase and glucokinase.

In this experiment, over 10% of the metabolic capability of the cells initially appeared to be directed toward secreted hexose production. Total hexose in the medium increased up to day 2 and then leveled off. On day 2, the total dry weight of the engineered cyanobacteria was about 280 µg/ml, and the total mass of hexose in the medium was about 33 µg/ml. Further, Figure 7 shows the exponential growth rates of the four strains, and illustrates that the strain carrying neither transgene grew the fastest (0.80 ± 0.002 per day), the strain carring the *glf* gene grew slightly more slowly (0.74 ± 0.01 per day), and the strains carrying the invertase (0.61 ± 0.01 per day) or the invertase gene and the *glf* gene grew the slowest (0.70 ± 0.04 per day), but these growth rates were not dramatically different. This indicates that under the conditions employed, sugar production did not lead to a major diversion of the carbon flux.

Figure 8 shows the amount of sucrose produced by the engineered cyanobacteria. The levels of sucrose in the medium were low during the initial phase of the experiment. Figure 8 shows that the extracellular sucrose was not detected in strains expressing *invA* only, or a combination of *invA* and *glf,* and there were not significant differences between the wild-type and *glf*-expressing *Synechococcus* strains.

Further, the combined extracellular and intracellular sucrose concentration was measured in the engineered cyanobacteria by sonicating a sample of the whole culture on day 3, including supernatant, prior to removal of cell debris by centrifugation. Figure 8 shows that no sucrose was observed from invertase-expressing strains. The amount of sucrose inside the wild-type and *glf*-expressing *Synechococcus* strains significantly exceeded the amount in the medium as compared with Figure 8. In addition, the moles of fructose in the medium secreted by the engineered cyanobacteria significantly exceeded the total moles of sucrose found in the wild-type strain and in the respective medium, as compared with Figure 6B.

Cyanobacterial cultures maintained with alternating dark and light periods accumulate polysaccharides when they are exposed to light and mobilize this intracellular reserve material in the dark. Smith, 134B Ann. Microbiol. 93 (1983). To address whether induced sugar synthesis occurs during the day or whether stored energy reserves are used during the night, the growth and fructose and glucose sugar secretion of *Synechococcus* expressing glf and *invA* were monitored as a function of the day-night cycle consisting of alternating 12 hours of light and 12 hours of darkness. Increases in the cell mass and net hexose sugar secretion occurred only in the presence of light; both parameters decreased in the dark (Figures 9A and 9B). This observation indicates that sucrose is synthesized during the day, and illustrates how engineered secretion systems can be used to noninvasively assay metabolic states.

In this Example, the following conditions and methods were used for culture of S. *elongatus* 7942. All *Synechococcus* strains were cultured at 30°C in BG-11 medium (Allen et al., 51 J. Gen. Microbio. 199 (1968)), the formula for which is available from the American Type Culture Collection (ATCC, Manassas, VA). Wild-type S. *elongatus* 7942 was obtained from the ATCC. Unless indicated otherwise, cultures were grown with a light cycle consisting of 18 hr of light and 6 hr of darkness. To select for homologous recombination and integration of heterologous DNA into the genome and for culture maintenance of engineered strains, kanamycin (25 µg/ml or 2.5 µg/ml if in combination with other drugs), spectinomycin (25 µg/ml or 2µg/ml if in combination with other drugs) was added to the medium. For all experiments, *Synechococcus* cultures were tested for possible contamination by spotting cultures onto LB medium plates; only data obtained with uncontaminated cultures are reported herein. Alternative concentrations of antibiotic concentrations, when used singly, were 50 µg/ml for kanamycin and 40 µg/ml for spectinomycin.

For sugar production experiments, liquid cultures were inoculated with exponentially growing cells (e.g. cells from a 2-3 day old dense culture) at an optical density of 750nm (OD₇₅₀) of 0.05. Sugar production was induced with 200 mM NaCl and 100 µM IPTG. Alternative concentrations of NaCl for induction of sugar production range between 100 to 300 mM (in Example 2). Growth was monitored by measuring the OD₇₅₀. Sugar production was determined using a sucrose-D-fructose-D-glucose assay kit (catalogue number KSUFRG; Megazyme, Ltd., Bray, Ireland). Assays were performed using culture supernatants prepared by centrifuging samples for 5 min at 21,130 x *g*. Assays were performed in triplicate, and standard deviations were determined.

### Example 2. Production of hexoses as a function of salt concentration.

As the NaCl concentration determines how much sucrose accumulates inside the cells (Li et al., 24 Biotech. Prog. 815 (2008)), the effect of different salt concentrations on hexose sugar secretion by *invA*+*glf*-expressing *Synechococcus* was determined. Table 1 shows growth rates of a *Synechococcus* empty vector control and *glf*+*invA* strain, and sugar concentrations in the culture medium four days after induction with 100 mM IPTG and various different NaCl concentrations in BG-11 medium. Figures 10A-10C are graphical representations of data shown in Table 1.

**Table 1: Effect of NaCl concentration on the growth rate and hexose sugar secretion**

| NaCl [mM] | growth rate [d⁻¹] | Sugar concentration [µM] | |
|---|---|---|---|
| | | Glucose | fructose |
| control | | | |
| 0 | 0.91 ±0.03 | 0.1 ±0.1 | 0.7 ±0.2 |
| 100 | 0.84 ±0.03 | 1.1 ±0.5 | 0.9 ±0.3 |
| 200 | 0.79 ±0.02 | 0.7 ±0.4 | 2.3 ±1.9 |
| 300 | 0.71 ±0.01 | 1.4 ±0.2 | 0.7 ±0.2 |

| *glf+invA* | | | |
|---|---|---|---|
| 0 | 0.88 ±0.001 | 2.2 ±2.5 | 0±0.6 |
| 100 | 0.79 ±0.02 | 8.6 ±6.3 | 25.1 ±2.0 |
| 200 | 0.71 ±0.02 | 31.3 ±3.6 | 144.4 ±18.2 |
| 300 | 0.53 ±0.01 | 40.0±1.5 | 128.9 ±4.5 |

With increasing salt concentrations, growth rates of the engineered strain decreased, and the inhibitory effect on growth of the *glf*+*invA*-expressing strain was more pronounced than the inhibition observed with a control strain (Table 1 and Figure 10A). In other experiments, use of 600 mM NaCl resulted in complete inhibition of growth of the *glf+invA-*expressing strain. The amounts of sugar produced were also dependent on the salt concentration, and highest for 200-300 mM NaCl (Table 1 and Figure 10B). Further, Figure 10C illustrates that increasing NaCl concentrations up to 300 mM resulted in an increasing level of hexose secretion, as calculated on a per cell basis.

On day 2 after induction of sugar secretion by 300 mM NaCl, sugar yield was about 30% of the biomass produced by the corresponding empty vector control under identical growth conditions.

### Example 3. Utilization of cyanobacterially secreted hexoses by a second bacterium.

In some metabolic engineering applications, it is useful to perform some metabolic transformations in one organism and other metabolic transformations in a second. For example, it is relatively less convenient to engineer cyanobacteria for certain metabolic transformations than to use, for example, *E. coli.* The cost of the carbon source in commercial fermentation (e.g., *E. coli)* is significant: as much as 30% to 50% of the overall operating cost (Galbe et al., 108 Adv. Biochem. Eng. Biotech. 303 (2007)). Therefore, the ability of hexose-secreting cyanobacteria to support growth *of E. coli* can be valuable.

Typical results are shown in Figures 11A-11E. About 10⁶ cells/ml of a prototrophic strain of *E*. *coli* that expressed the yellow fluorescent protein (YFP) were added to cultures of a glf+invA-expressing strain of *S. elongatus* 7942 or a control strain at an OD₇₅₀ of 0.1. At the time of addition of the *E. coli,* 100 micromolar IPTG and 200 mM NaCl were also added to induce hexose secretion. Figure 11A illustrates that both cyanobacterial strains continued to grow in the presence of the *E. coli.* Figures 11B-11C illustrates that the *E. coli* were able to increase in number in the presence of the *glf*+*invA*-expressing strain of *S*. *elongates* 7942, but not in the presence of the control strain of *S*. *elongatus* 7942. These results indicate that nutrient-secreting cyanobacteria can be used to support the metabolism of a second microbe (e.g., *E. coli)* without addition of an external carbon source in liquid culture (Figures 11B and 11C) or on solid medium (Figures 11D and 11E). Without wishing to be bound by theory, coculture of sugar-secreting cyanobacteria and a second engineered microbe can allow production of a desired product without a reduced-carbon feedstock in situations where synthesis of the product is incompatible with cyanobacterial metabolism.

*Experimental methods:* Cultures of the *Synechococcus glf+invA* strain and a non-sugar-secreting control strain were grown to an OD₇₅₀ of 0.1. Sugar secretion was induced with 200 mM NaCl and 100 µM IPTG. *E. coli* cells from an overnight culture (LB + Kan, Spec + 100 µl IPTG) were washed in PBS three times and incubated with shaking in PBS containing 100 µM IPTG and antibiotics for 1 hr at 37°C, after which 10⁶ cells/ml (30µl) were added to 30 ml of either *glf+invA* or the empty vector control *Synechococcus* cultures. The medium used was BG-11 medium with 100 µM IPTG, 2.5 µg/ml kanamycin, 2 µg/ml spectinomycin, and 1 mg/ml NH₄Cl. The *E. coli* strain carried a plasmid that was a hybrid of the pET47b (Kan), with a Spectinomycin-Resistance gene and a promoter driving expression of YFP. Thus, the *E. coli* was also resistant to antibiotics used in the culture. Growth *of E. coli* was monitored by plating serial dilutions on LB agar and measuring the YFP fluorescence of culture samples with a Victor 3V plate reader. For microscopic quantitation of *Synechococcus* and *E. coli,* bacteria in samples of the liquid coculture were visualized by red chlorophyll autofluorescence and YFP fluorescence, respectively. To observe microcolony formation, 100 µl of the initial coculture was plated on BG-11 agar containing the compounds listed above. After four days, pieces of agar were cut out and placed onto a MatTek glass bottom dish for microscopy.

### Example 4. Production of lactic acid from a photosynthetic microbe.

Using a similar approach to that was described in Example 1 for engineering of a photosynthetic bacterium to produce hexose sugars, a cyanobacterial strain was engineered to produce lactic acid. Specifically, lactate dehydrogenase *ldhA* from *E. coli* K12 (Plumbridge, 33 Mol. Microbio. 260 (1999)) was inserted into the DS21 plasmid, and the D,L-lactate transporter gene *lldP* from E. *coli* K12 (Nunez et al., 290 Biochem. Biophys. Res. Commun. 824 (2002)) was inserted into the DS 1321 plasmid. Diagrams of these plasmid vectors for expression of *ldhA* and *lldP* in *S. elongatus* are shown in Figures 12A-C, and the sequences of the inserted *ldhA* and *lldP* genes with their respective amino acid sequences are shown in Figures 13A-B and 14A-B, respectively.

*Synechococcus* naturally produces pyruvate as a metabolic intermediate. The *E. coli ldhA* gene encodes lactate dehydrogenase, which catalyzes the reduction of pyruvate to lactate (Plumbridge, 1999), and the E. *coli lldP* gene encodes a lactate transporter protein in the major facilitator superfamily. DiMarco et al., 1985; Snoep et al., 1994. The *LldP* protein cotransports lactate with a proton (Nunez et al., 2002); during *Synechococcus* growth in BG-11 medium, when the culture reaches an OD₇₅₀ of 1, the pH of the medium is generally about pH 9, so lactate will be exported from the cell if it is produced intracellularly. The schematic depiction of this engineering scheme is illustrated in Figure 15.

The *ldhA* and *lldP* were obtained by PCR amplification from E. *coli* DH5α using oligonucleotides that resulted in molecules with an N-terminal His₆ tag. To verify the activity of transgenes in the engineered cyanobacteria, expression of *LdhA-*His₆ in *Synechococcus* was observed by western blotting, while *LldP*-His₆ was not detected (Figure 5B), perhaps due to low levels of expression combined with poor extraction from membranes. Expression of *ldha* in *Synechococcus* resulted in increased levels of intracellular lactate.

The resulting derivative of *S*. *elongatus* 7942 was tested along with various controls for the production of D-lactic acid. Lactate production was determined in the supernatant of the culture, which was spun down for 5 min at 21,130 x g, using an LD-lactic acid detection kit from R-Biopharm (Marshall, MI) adapted for a 96-well plate reader (Victor 3V; Perkin-Elmer) by using 1/10 of the amounts of the reagents recommended by the manufacturer in each assay mixture.

Typical results are shown in Figures 17A-17B. Analogous to *Synechococcus* cells engineered to secrete glucose and fructose in Example 1, *Synechococcus* ells expressing the *E. coli ldhA* and *lldP* genes from IPTG-inducible promoters secreted relatively high levels of lactate into the medium, while cells expressing only *ldhA* or *lldP* produced ∼4-fold-lower levels (Figure 16A), and there was no dramatic difference in the growth rates of these four strains (Figure 16B). The rate of accumulation of extracellular lactate was much lower than the rate of accumulation of hexose sugars, but it increased steadily for at least 9 days. These observations indicate that the net intracellular rate of lactate production is lower than the net intracellular rate of glucose and fructose production but that after secretion lactate cannot be taken up and metabolized.

### Example 5. Enhanced production of lactic acid by additional engineering

The production of lactic acid is enhanced by the expression of certain additional transgenes. For example, expression of a lactate operon from *Lactococcus lactis* leads to the production of L-lactic acid. This enantiomer of D-lactic acid is also transported outside the cell by the *lldP* transporter. This lactate operon includes the genes Lactate dehydrogenase, Pyruvate Kinase and Phosphofructokinase.

Photosynthetic microbes (e.g., *S. elongatus* 7942) produce NADPH as the major carrier of reducing equivalents, but lactate dehydrogenase uses NADH as its reducing substrate (Figure 17A). As an alternative or additional method for enhancing lactic acid production, the engineered *S. elongatus* 7942 strain in Example 4 that expressed the *ldhA* and *lldP* genes of *E. coli* was further engineered to also express the *udhA* gene *of E. coli,* which encodes a soluble NADPH/NADH transhydrogenase (Boonstra et al, 181 J. Bacteriol. 1030 (1999)). The IPTG-inducible *lac* promoter was used.

Production of lactic acid from the resulting strain was compared to production of lactic acid from a corresponding strain expressing only the *ldhA* and *lldP* genes *of E. coli* and containing an empty version of the pHN1-LacUV5 plasmid (Figure 17B). The diagram of the plasmid with udhA gene insertion is shown in Figure 17C. Upon induction of all transgenes with IPTG, lactate levels in culture supernatant was markedly enhanced and reached 600 micromolar from the *ldhA, lldP, udhA* strain of *S. elongatus* 7942 after four days, while levels of lactic acid in culture supernatant of the *ldhA, lldP* strain of S. *elongatus* 7942 accumulated to <100 µM (Figure 17B). In addition, the *ldhA, lldP, udhA* strain of *S. elongatus* 7942 grew extremely slowly after induction with IPTG. This might be due to a flux of energy and carbon into lactate, to alterations in the intracellular pH, or to lower levels of NADPH impacting the regulatory protein OpcA and activating the oxidative pentose phosphate pathway (Hagen et al., 276 J. Biol. Chem. 11477 (2001)). More importantly, the reduction in the growth rate of engineered *Synechococcus* further expressing the *udhA* transhydrogenase indicates that on a per cell basis lactic acid by the *ldhA, lldP, udhA* strain was particularly high compared to its parent strain. Moreover, the initial rate of production of lactate in the strain expressing lactate dehydrogenase, the lactate transporter, and NADP/NAD transhydrogenase was about 54 mg/liter/day/OD₇₅₀ unit (Figure 17B).

Mutations in other genes to reduce or eliminate the following enzymes also enhances lactate production: malate dehydrogenase, pyruvate dehydrogenase complex, phosphoenolpyruvate carboxylase, phosphoenolpyruvate synthase.

*Plasmid and vector construction:* To express additional genes for pathway optimization, the *udhA* gene was introduced into a distinct plasmid integrating vector, termed pHN1-LacUV5, which was designed to integrate at a distinct NS 3 in the S. *elongates* 7942 genome (Figures 19A-19B). The vector expresses *E. coli lacI and* encodes chloramphenicol resistance, mediates integration into the remnant of a cryptic prophage in the *Synechococcus* genome, and contains a *lac* operon promoter followed by a multiple-cloning site. To select for homologous recombination and integration of heterologous DNA into the genome and for culture maintenance of engineered strains, kanamycin (25 µg/ml or 2.5 µg/ml if in combination with other drugs), spectinomycin (25 µg/ml or 2 µg/ml if in combination with other drugs) and chloramphenicol (12.5 µg/ml or 2.5 µg/ml if in combination with other drugs) was added to the medium.

### Example 6. Enhanced production of hexose sugars by additional metabolic engineering

To further demonstrate that secretion of hydrophilic products of interest from *Synechococcus* could be further enhanced by increasing the levels of intracellular precursors, heterologous enzymes were expressed to improve the production of intracellular sucrose. Without wishing to be bound by theory, the rationale was as follows. Sucrose is normally synthesized from fructose-1-phosphate and UDP-glucose, which are condensed to form sucrose phosphate that is then dephosphorylated to generate sucrose (Figure 19A). UDP-glucose is synthesized by UDP-glucose phosphorylase, encoded by the *E. coli galU* gene. Marolda et al., 22 Mol. Microbiol 827 (1996). Integration of the *galU* gene, encoding UDP-glucose phosphorylase, expressed from an IPTG-regulated promoter at a distinct *Synechococcus* NS 3 using the pHN1-LacUV5 vector further increased hexose sugar production by more than 30% in cells expressing *invA* and glf (Figure 19B). Figure 19C shows the diagram of the plasmid of *galU* insertion in *Synechococcus.*

The market value of commodity organic compounds, such as sugars, lactic acid and amino acids is generally higher than the actual market value of fuels on a per-photon basis. Therefore, engineering photosynthetic microbes to produce and secrete hydrophilic or charged molecules is economically desirable. In addition, biofuels are usually produced by fermentation of sugars. Engineering photosynthetic microbes to produce and secret hydrophilic compounds, such as sugars, contemplates a cost-effective method for biofuel production.

### Example 7. Expression of a sucrose/H⁺ symport protein in cyanobacteria.

In some embodiments of the present invention use proton symport proteins as transporters for the secretion of metabolites from cyanobacteria. Without wishing to be bound by theory, this is because cyanobacteria tend to alkalinize their growth medium so that when a metabolite is co-transported out of the cell with an H⁺ ion, such transport is essentially irreversible.

To further illustrate this principle, a cyanobacterium such as *Synechococcus elongatus* PCC 7942 or *Synechocystis* species PCC 6803 is engineered to express the sucrose/H⁺ symporter CscB. The CscB protein is similar to LacY permease for lactose (Vadyvaloo et al., 358 J. Mol. Bio. 1051 (2006)); this protein is localized to the plasma membrane, has about twelve transmembrane α-helices, and key amino acids involved in cation co-transport are conserved between the LacY permease and the CscB protein. The nuclei acid and amino acid coding sequences for the CscB protein of *E*. *coli* are given in Figures 20A and 20B, respectively.

The CscB-encoding sequence is placed in an expression vector for S. *elongatus* PCC7942 in essentially the same manner as described above for the *glf* or *lldP* coding sequences as described above. The promoter used is an inducible promoter such as a derivative of the *E. coli lac* promoter as described above, or a NaCl-inducible promoter. Alternatively, a constitutive promoter can be used. The resulting CscB expression vector is introduced into *S. elongatus* PCC7942 by standard transformation procedures known to one of skill in the art. The resulting S. *elongatus* PCC7942 strain expresses a functional CscB protein.

Evidence for functionality of the CscB protein is demonstrated, for example, as follows. Cyanobacteria expressing the CscB protein can grow very slowly in the absence of light when sucrose is present and the pH is buffered to about pH 7. Concomitant expression of invertase is optionally used to demonstrate this point. Alternatively, when the engineered cyanobacteria are induced to generate intracellular sucrose with NaCl, sucrose is found in the extracellular medium at levels significantly higher than the levels of sucrose in the medium of NaCl-induced, non-engineered S. *elongatus* PCC7942. For example, when a culture of CscB-expressing S. *elongatus* PCC7942 at an OD of about 0.05 are induced with 200 mM NaCl, levels of at least about 50 µM sucrose accumulate in the extracellular medium within 2 days. In comparable cultures of non-engineered *S. elongatus,* the amount of sucrose in the extracellular medium is less than one-half of the levels found in the cultures of the CscB-expressing recombinant *S*. *elongatus.*

Production of sucrose may be increased further by expression of enzymes involved in sucrose synthesis, such as sucrose phosphate synthase plus sucrose phosphate phosphatase, and/or such as UDP-glucose phosphorylase as described herein.

### Example 8. Engineered synthesis and secretion of amino acids from a photosynthetic microbe.

Glutamate is a commercially important amino acid, being used as a food additive in the form monosodium glutamate (MSG). To further illustrate aspects of the invention, a cyanobacterium such as *Synechococcus elongatus* PCC7942 or *Synechocystis* sp. PCC6803 is engineered to synthesize and secrete glutamate.

The glutamate transporter GltS promotes secretion of glutamate. GltS cotransports glutamate and Na⁺ ions. Sodium and proton gradients are coupled by a Na⁺/H⁺ antiporter, so that systems involving co-transport with Na⁺ are indirectly coupled to the proton gradient. Thus, metabolites produced by alkalinizing photosynthetic bacteria that are co-transported with Na⁺ will accumulate outside of the cell, just as metabolites co-transported with H⁺ will accumulate outside of the cell. The nuclei acid and amino acid coding sequences for the GltS protein *of E. coli* are given in Figures 21A and 21B.

The GltS-encoding sequence is placed in an expression vector for S. *elongatus* PCC7942 in essentially the same manner as described above for the *glf* or *lldP* coding sequences as described above. The promoter used is an inducible promoter such as a derivative of the *E. coli lac* promoter as described above, or a NaCl-inducible promoter. Alternatively, a constitutive promoter can be used. The resulting GltS expression vector is introduced into *S. elongatus* PCC7942 by standard transformation procedures known to one of skill in the art. The resulting S. *elongatus* PCC7942 strain expresses a functional GltS protein.

Evidence for functionality of the GltS protein is demonstrated, for example, as follows. When the recombinant cyanobacteria are induced to express GltS, glutamate accumulates in the extracellular medium. In comparable cultures of non-engineered S. *elongatus,* the amount of glutamate in the extracellular medium is less that one half of the levels found in the cultures of the recombinant, GltS-expressing S. *elongatus.*

The invention includes the following embodiments.
1. A recombinant photosynthetic bacterium comprising at least one heterologous enzyme and at least one heterologous transporter, wherein said transporter mediates the secretion of a product whose synthesis is enhanced by said enzyme.
2. The recombinant photosynthetic bacterium of embodiment 1, wherein the transporter is a member of the major facilitator superfamily.
3. The recombinant photosynthetic bacterium of embodiment 1, wherein the heterologous transporter encoded by a gene selected from the group consisting of: the Glut family of glucose transport proteins in humans and other mammals; the HXT family of glucose transporters in yeast; *lacY; lldP* lactate transporter of *E*. *coli* or another bacterium that performs anaerobic metabolism; a mammalian citric acid transport protein; the *glpT* glycerol phosphate transporter; and *GLF* of *Z. mobilis.*
4. The recombinant photosynthetic bacterium of embodiment 1, wherein the enzyme is an invertase.
5. The recombinant photosynthetic bacterium of embodiment 3 or 4, further comprising a reduction-of-function mutation in an endogenous hexokinase gene, an endogenous glucokinase gene, or both.
6. The recombinant photosynthetic bacterium of any of embodiments 1-5, wherein the product is a chiral compound.
7. The recombinant photosynthetic bacterium of embodiment 6, wherein the chiral compound is a hexose sugar.
8. The recombinant photosynthetic bacterium of embodiment 7, wherein the hexose sugar is glucose, fructose, or a mixture of glucose and fructose.
9. The recombinant photosynthetic bacterium of embodiment 1, wherein the enzyme is lactate dehydrogenase and the transporter is a lactate transporter.
10. The recombinant photosynthetic bacterium of embodiment 9, wherein the lactate transporter is encoded by the *lldP* gene of *E*. *coli.*
11. The recombinant photosynthetic bacterium of embodiment 9 or 10, further comprising a reduction-of-function mutation in malate dehydrogenase, pyruvate dehydrogenase complex, phosphoenolpyruvate carboxylase, phosphoenolpyruvate synthase.
12. The recombinant photosynthetic bacterium of any of embodiments 9-11, wherein the product is lactic acid.
13. The recombinant photosynthetic bacterium of any of embodiments 1-12, wherein the enzyme is expressed from an inducible promoter.
14. The recombinant photosynthetic bacterium of embodiment 13, wherein the photosynthetic bacterium is a cyanobacterium.
15. The recombinant photosynthetic bacterium of embodiment 14, wherein the cyanobacterium is selected from *Prochlorococcus spp., Synechococcus spp.,* and *Synchecocystis spp.*
16. The recombinant photosynthetic bacterium of any one of embodiments 1 to 15, wherein the photosynthetic bacterium is additionally engineered to express a soluble NAD(P) transhydrogenase.
17. The recombinant photosynthetic bacterium of any of embodiments 1 to 15, wherein the photosynthetic bacterium is additionally engineered to express a UDP-glucose phosphorylase.
18. A recombinant photosynthetic bacterium comprising at least one heterologous transporter, wherein said transporter mediates the symport or antiport of a product and an inorganic ion.
19. The recombinant photosynthetic bacterium of embodiment 18, wherein said inorganic ion is selected from the group consisting of a proton, a sodium ion, a potassium ion, a chloride ion, and a hydroxide ion.
20. The recombinant photosynthetic bacterium of embodiment 18, wherein said transporter is selected from the group consisting of a lactic acid/proton symporter, a sucrose proton symporter, a lactose/proton symporter, a dicarboxylic acid/proton symporter, an amino acid/proton symporter, a citrate/proton symporter, an amino acid/sodium ion symporter, and an amino acid/hydroxide ion antiporter.
21. The recombinant photosynthetic bacterium of embodiment 18, wherein the heterologous transporter encoded by a gene selected from the group consisting of: the sucrose transporter CscB; the lactose permease of E. coli encoded by the lacY gene; the mammalian H+/peptide transporter PepT1H; the glutamate transporter GltS and the lactate transporter *lldP.*
22. The recombinant photosynthetic bacterium of embodiment 18, wherein the product is a chiral product.
23. The recombinant photosynthetic bacterium of embodiment 18, wherein the product is an amino acid.
24. The recombinant photosynthetic bacterium of embodiment 23, wherein the amino acid is glutamate.
25. The recombinant photosynthetic bacterium of embodiment 18, wherein the product is a sugar.
26. The recombinant photosynthetic bacterium of embodiment 18, further comprising at least one more heterologous gene.
27. The photosynthetic bacterium of embodiment 26, wherein the heterologous gene encodes an enzyme or a transporter.
28. The photosynthetic bacterium of embodiment 27, wherein the enzyme is *ldhA.*
29. A method for producing a product, said method comprising culturing a recombinant photosynthetic bacterium of any of embodiments 1 to 17 in the presence of light and carbon dioxide, and obtaining the product from the supernatant of the cultured bacterium.
30. A method for producing a product, said method comprising culturing a recombinant photosynthetic bacterium of any of embodiments 18-28 in the presence of light and carbon dioxide, and obtaining the product from the supernatant of the cultured bacterium.

## Claims

1. A recombinant photosynthetic bacterium comprising at least one heterologous transporter, wherein the heterologous transporter mediates the symport or antiport of sucrose and a cation.

2. The recombinant photosynthetic bacterium of claim 1, wherein the cation is a proton.

3. The recombinant photosynthetic bacterium of claim 1 or 2, wherein the heterologous transporter is a sucrose proton symporter.

4. The recombinant photosynthetic bacterium of claim 3, wherein the surcrose proton symporter is encoded by the gene CscB.

5. The recombinant photosynthetic bacterium of any of preceding claims, further comprising at least one more heterologous gene.

6. The recombinant photosynthetic bacterium of claim 5, wherein the heterologous gene encodes an enzyme or a transporter.

7. The recombinant photosynthetic bacterium of any of preceding claims, wherein the heterologous transporter is expressed from a promoter.

8. The recombinant photosynthetic bacterium of claim 7, wherein the promoter is selected from the group consisting of inducible promoters; promoters that are naturally associated with transposon- or bacterial chromosome-borne antibiotic resistance genes; promoters of heterologous bacterial or native cyanobacterial genes; promoters from viruses or phages; and synthetic promoters.

9. The recombinant photosynthetic bacterium of claim 7, wherein the promoter is a promoter that is inducible by addition of isopropyl β-D-1-thiogalactopyranoside (IPTG).

10. The recombinant photosynthetic bacterium of any of preceding claims, wherein the photosynthetic bacterium is additionally engineered to express an enzyme involved in sucrose synthesis.

11. The recombinant photosynthetic bacterium of claim 10, wherein the enzyme involved in sucrose synthesis is selected from the group consisting of sucrose phosphate synthase, sucrose phosphate phosphatase, UDP-glucose phosphorylase, and any combinations thereof.

12. The recombinant photosynthetic bacterium of any preceding claim, wherein the photosynthetic bacterium is a cyanobacterium.

13. The recombinant photosynthetic bacterium of claim 12, wherein the cyanobacterium is selected from *Prochlorococcus spp., Synechococcus spp.,* and *Synchecocystis spp.*

14. A method for producing sucrose comprising culturing a recombinant photosynthetic bacterium of any of claims 1-13 in a culture medium in the presence of light and carbon dioxide, and obtaining the sucrose form the supernatant of the cultured bacterium.

15. The method of claim 14, further comprising inducing the recombinant photosynthetic bacterium to generate intracellular sucrose.

16. The method of claim 15, wherein the inducing is performed by addition of salt into the culture medium.

17. The method of any preceding claim, further comprising inducing expression of the heterologous transporter in the recombinant photosynthetic bacterium.

18. The method of claim, wherein the culture medium is alkaline.


SEQUENCE LISTING

<110> PRESIDENT AND FELLOWS OF HARVARD COLLEGE

<120> PRODUCTION OF SECRETED BIOPRODUCTS FROM PHOTOSYNTHETIC MICROBES

<130> 002806-067641-PCT

<140> PCT/US2010/047830
<141> 2010-09-03

<150> 61/317,001
<151> 2010-03-24

<150> 61/239,985
<151> 2009-09-04

<160> 14

<170> PatentIn version 3.5

<210> 1
<211> 7041
<212> DNA
<213> Artificial Sequence

<220>
<223> Description of Artificial Sequence: Synthetic
      polynucleotide

<400> 1
gatccggcag ccggcggagc gctgctttct tggcaagcgg tcgccagccc caacgccagg      60

gctgccagcc cgaaacagcg gggcaaggca gcttggaagg gcgatcgcag cacgggcatg     120

gcaatgtctc tctgaaggaa tgcagacctt attcgtacag ccagggttga atcgtggggg     180

tccaatcact tagctctgct gggctaaacc agagagcaat ttcctgttgt gctgtttcga     240

ttgcatccga gccatggatg atgttgcggc caatattgac accaaaatca ccacggatgg     300

tgcccggttc tgccgtcagc ggattggtag cgccgatcaa cttgcgagca gccgccacaa     360

cgccttcgcc ttccaagacg atcgccacga tcggcccaga ggtgatgaac tcgacgaggc     420

cattgaagaa ggggcgctcg cggtggacag catagtgctg ttcggccagc tcgcgactgg     480

gcttcagctg ctttaggccc accagtttga agcctttttg ctcaaagcgg ccgatgatcg     540

taccgaccaa accccgctga acgccatcgg gcttgatggc aataaatgtg cgttccacag     600

acatctagat agtcctcaag acgaggcaag cattgagctt gccttcctat ggttcgggat     660

cactgggatt cttgacaagc gatcgcggtc acatcgctat ctcttaggac ttcgcagcgg     720

gcgagtcgga ttgacccggt agggatttcg ccagatcaat gcccgtggtt tgtttcagct     780

tctccagcaa gctagcgatt tgggtagcgc tgccttcccc ttcgccaatc acagtgatcg     840

actccacgtc gatatctggc acggtgcctg aaagcgtgac gagcagggac tcgaagcttg     900

catgcctgca ggtcgactct agagctttat gcttgtaaac cgttttgtga aaaaattttt     960

aaaataaaaa aggggacctc tagggtcccc aattaattag taatataatc tattaaaggt 1020

cattcaaaag gtcatccacc ggatcaattc ccctgctcgc gcaggctggg tgccaagctc 1080

tcgggtaaca tcaaggcccg atccttggag cccttgccct cccgcacgat gatcgtgccg 1140

tgatcgaaat ccagatcctt gacccgcagt tgcaaaccct cactgatccg catgcccgtt 1200

ccatacagaa gctgggcgaa caaacgatgc tcgccttcca gaaaaccgag gatgcgaacc 1260

acttcatccg gggtcagcac caccggcaag cgccgcgacg gccgaggtct tccgatctcc 1320

tgaagccagg gcagatccgt gcacagcacc ttgccgtaga agaacagcaa ggccgccaat 1380

gcctgacgat gcgtggagac cgaaaccttg cgctcgttcg ccagccagga cagaaatgcc 1440

tcgacttcgc tgctgcccaa ggttgccggg tgacgcacac cgtggaaacg gatgaaggca 1500

cgaacccagt ggacataagc ctgttcggtt cgtaagctgt aatgcaagta gcgtatgcgc 1560

tcacgcaact ggtccagaac cttgaccgaa cgcagcggtg gtaacggcgc agtggcggtt 1620

ttcatggctt gttatgactg tttttttggg gtacagtcta tgcctcgggc atccaagcag 1680

caagcgcgtt acgccgtggg tcgatgtttg atgttatgga gcagcaacga tgttacgcag 1740

cagggcagtc gccctaaaac aaagttaaac atcatgaggg aagcggtgat cgccgaagta 1800

tcgactcaac tatcagaggt agttggcgtc atcgagcgcc atctcgaacc gacgttgctg 1860

gccgtacatt tgtacggctc cgcagtggat ggcggcctga agccacacag tgatattgat 1920

ttgctggtta cggtgaccgt aaggcttgat gaaacaacgc ggcgagcttt gatcaacgac 1980

cttttggaaa cttcggcttc ccctggagag agcgagattc tccgcgctgt agaagtcacc 2040

attgttgtgc acgacgacat cattccgtgg cgttatccag ctaagcgcga actgcaattt 2100

ggagaatggc agcgcaatga cattcttgca ggtatcttcg agccagccac gatcgacatt 2160

gatctggcta tcttgctgac aaaagcaaga gaacatagcg ttgccttggt aggtccagcg 2220

gcggaggaac tctttgatcc ggttcctgaa caggatctat ttgaggcgct aaatgaaacc 2280

ttaacgctat ggaactcgcc gcccgactgg gctggcgatg agcgaaatgt agtgcttacg 2340

ttgtcccgca tttggtacag cgcagtaacc ggcaaaatcg cgccgaagga tgtcgctgcc 2400

gactgggcaa tggagcgcct gccggcccag tatcagcccg tcatacttga agctagacag 2460

gcttatcttg gacaagaaga agatcgcttg gcctcgcgcg cagatcagtt ggaagaattt 2520

gtccactacg tgaaaggcga gatcaccaag gtagtcggca aataatgtct aacaattcgt 2580

tcaagccgac gccgcttcgc ggcgcggctt aactcaagcg ttagatgcac taagcacata 2640

attgctcaca gccaaactat caggtcaagt ctgcttttat tatttttaag cgtgcataat 2700

aagccctaca caaattggga gatatatcat gaaaggctgg ctttttcttg ttatcgcaat 2760

agttggcgaa gtaatcgcaa catccgcatt aaaatctagc gagggcttta ctaagctgat 2820

ccggtggatg accttttgaa tgacctttaa tagattatat tactaattaa ttggggaccc 2880

tagaggtccc ctttttattt ttaaaaattt tttcacaaaa cggtttacaa gcataaagct 2940

ctagaggatc ggcggccgca agctttctag attagtggtg gtggtggtgg tggctgctgc 3000

cgcaggcatc gctgctaaag gcggccaggt tggcatcttg cagggtccag cgtttaaagc 3060

tatcaaacac ggcgtagcct tcgatggcaa acaggcggcc tttcaggcta tctttatcgg 3120

gaaagatgcg gctgctgatg ctgtacaggc cctgggtctg atcatcgccc acaaagattt 3180

cgatgctgct gcgatccaga aagatgtgca ggcgcacttt gctggtgttg ggcagggggc 3240

agctgcggat gcctttcacg ttctggccgc tgcggttgcg atccaggatg atgcggttct 3300

ggctgcgatc gatgtacagc agggtttcct gacccttacc gttgcagcgc agggccaggc 3360

cagcctggta agcgctgctc ttttccagat caaagatcag ttcgatttct tgcagggggc 3420

tatccatggt aaaggggtgt tcggcatcgc tcagggtcac cacgctttcg atcttttcgc 3480

tctggcgcag gatttccatt tcgcgcacgg gggtcatcac gatttgttta tcgatcagat 3540

ccagtttgcg gggcagggtc atgcagccgg cccagccatc gcgctggctg ggtttctggt 3600

tttcccacat atcaaaccag gcgatcagga tctggcggcc atctttggct tcaaagcgct 3660

gggcggcgta aaaatcgtgg ccgtaatcca gttcctgaaa gctggtttcg ggggtaaact 3720

ggggggcctg ccatttgccc aggatgtagc cgttctgaaa caggttgcgg tttttgtagc 3780

cgctggcttt caggccctgg gggctaaaca tcagcacgct gcggttgccc aggctaaaga 3840

aatcggggca ttcccacata aaggcgcgtt tgcccagggg cagctggctg ttatcgccca 3900

gcagggtttt cacaaagatc caatctttca ggttttcgct gcggtacagg gccacgtggc 3960

cgatgccctg gtgtttttca tcatcggtgc ggtagcccac caccataaac cagtggttgt 4020

tttctttcca cacgcgggga tcgcgaaagt gggccacctg gggcatgggg gctttttcca 4080

gcacgatgcc ttctttctga aagtggatgc catcgatgct ggtcgccatg cactgcactt 4140

cgcggatggc atccaggcta tcgttgctca gcacgatgtg gccggtgtag atcagggtca 4200

gcacgccgtt gttatccacg gcgcagccgc taaagcagcc atcgcgatca aagctatcgc 4260

cgggggccag ggccactggc agggtttccc agtgcaccag atcgcggctt ttagcgtgac 4320

cccagtgcat tggaccccac actggagcaa aggggtagta ctggtaaaac aggtggtatt 4380

cgcctttgaa aaagatcagg ccgttgggat cgttcatcca gccggtcagg ggggtcacgt 4440

gaaagccggg gtaccattcg ctgctcagca ggcgtttgcc ggctttcttc tgggcatctt 4500

cggctttgat caggtttttg tagctggggc tttcactaga catttttttt tcctccttgt 4560

gtgaaattgt tatccgctca caattccaca cattatacga gccggatgat taattgtcaa 4620

cagctcattt cagaatattt gccagaaccg ttatgatgtc ggcgcaaaaa acattatcca 4680

gaacgggagt gcgccttgag cgacacgaat tatgcagtga tttacgacct gcacagccat 4740

accacagctt ccgatggctg cctgacgcca gaagcattgg tgcaccgtgc agtcgatgat 4800

aagctgtcaa accagatcaa ttcgcgctaa ctcacattaa ttgcgttgcg ctcactgccc 4860

gctttccagt cgggaaacct gtcgtgccag ctgcattaat gaatcggcca acgcgcgggg 4920

agaggcggtt tgcgtattgg gcgccagggt ggtttttctt ttcaccagtg agacgggcaa 4980

cagctgattg cccttcaccg cctggccctg agagagttgc agcaagcggt ccacgctggt 5040

ttgccccagc aggcgaaaat cctgtttgat ggtggttaac ggcgggatat aacatgagct 5100

gtcttcggta tcgtcgtatc ccactaccga gatatccgca ccaacgcgca gcccggactc 5160

ggtaatggcg cgcattgcgc ccagcgccat ctgatcgttg gcaaccagca tcgcagtggg 5220

aacgatgccc tcattcagca tttgcatggt ttgttgaaaa ccggacatgg cactccagtc 5280

gccttcccgt tccgctatcg gctgaatttg attgcgagtg agatatttat gccagccagc 5340

cagacgcaga cgcgccgaga cagaacttaa tgggcccgct aacagcgcga tttgctggtg 5400

acccaatgcg accagatgct ccacgcccag tcgcgtaccg tcttcatggg agaaaataat 5460

actgttgatg ggtgtctggt cagagacatc aagaaataac gccggaacat tagtgcaggc 5520

agcttccaca gcaatggcat cctggtcatc cagcggatag ttaatgatca gcccactgac 5580

gcgttgcgcg agaagattgt gcaccgccgc tttacaggct tcgacgccgc ttcgttctac 5640

catcgacacc accacgctgg cacccagttg atcggcgcga gatttaatcg ccgcgacaat 5700

ttgcgacggc gcgtgcaggg ccagactgga ggtggcaacg ccaatcagca acgactgttt 5760

gcccgccagt tgttgtgcca cgcggttggg aatgtaattc agctccgcca tcgccgcttc 5820

cactttttcc cgcgttttcg cagaaacgtg gctggcctgg ttcaccacgc gggaaacggt 5880

ctgataagag acaccggcat actctgcgac atcgtataac gttactggtt tcacattcac 5940

caccctgaat tgactctctt ccgggcgcta tcatgccata ccgcgaaagg ttttgcacca 6000

ttcgatggtg tcaacgtaaa tgcatgccgc ttcgccttcg cgcgcgaatt gatctgctgc 6060

ctcgcgcgtt tcggtgatga cggtgaaaac ctctgacaca tgcagctccc ggagacggtc 6120

acagcttgtc tgtaactagt gggcccccta gggaattctc gaggcctact tgtggatccc 6180

cgggtaccga gctcgaattt cgagcttctg gagcaggaag atgtcgcggg cattagcacc 6240

agcggtctgc caagcctccg ccagccgttg ggtcccttcc gcttgagctt ttccatcttc 6300

gacgatacgg gcggcggccc cccgcgcttc cgcgatcgcc cgtttacaag ctgcctcagc 6360

tggggcgatc acatcggctt gaagttgctg ctgcacctgt ttgatccgct cctgctgcac 6420

agggagttct gcttggctac gagcgacttc ggtagcaatg tccgcttcag cttcggccac 6480

caccgcttcg cgccgcgtca acgcatcctg aatccggcgc tcggcctcgg cttgggcgat 6540

cgctacatcg cgatcgatcc gacgcagggc cgtgatcttg tcattttcgg ccgtttggat 6600

cgcagaggca gcctgggcat cggcttcagc aattcgggca tctcgctgca gatcagcccg 6660

ctgcttgcgt ccactagccg agagataacc gacctcatcg gaaatgttct ggacttgcag 6720

cgtatcgagg actagaccca gctgctcaag gtcatcctcc gcctcttcca gcagactttt 6780

ggcaaaggca attttgtcct cgttgatctg ctccggcgtg aggctggcta aaacaccacg 6840

caagttgcct tcgagggtct ccttggcaat ttgctcgatt tccttacggt ttttgccaag 6900

cagccgctcg atcgcgttgt ggatggtcgg ttcttcccca gcaatcttga tattggcaac 6960

gccttcaaca gtcaggggaa tgccgccctt ggagaaggca ttggaaacgc gcaactcaat 7020

gatcatgttg gtcagatcca t 7041

<210> 2
<211> 523
<212> PRT
<213> Artificial Sequence

<220>
<223> Description of Artificial Sequence: Synthetic
      polypeptide

<400> 2
Met Ser Ser Glu Ser Pro Ser Tyr Lys Asn Leu Ile Lys Ala Glu Asp
1               5                   10                  15

Ala Gln Lys Lys Ala Gly Lys Arg Leu Leu Ser Ser Glu Trp Tyr Pro
            20                  25                  30

Gly Phe His Val Thr Pro Leu Thr Gly Trp Met Asn Asp Pro Asn Gly
        35                  40                  45

Leu Ile Phe Phe Lys Gly Glu Tyr His Leu Phe Tyr Gln Tyr Tyr Pro
    50                  55                  60

Phe Ala Pro Val Trp Gly Pro Met His Trp Gly His Ala Lys Ser Arg
65                  70                  75                  80

Asp Leu Val His Trp Glu Thr Leu Pro Val Ala Leu Ala Pro Gly Asp
                85                  90                  95

Ser Phe Asp Arg Asp Gly Cys Phe Ser Gly Cys Ala Val Asp Asn Asn
            100                 105                 110

Gly Val Leu Thr Leu Ile Tyr Thr Gly His Ile Val Leu Ser Asn Asp
        115                 120                 125

Ser Leu Asp Ala Ile Arg Glu Val Gln Cys Met Ala Thr Ser Ile Asp
    130                 135                 140

Gly Ile His Phe Gln Lys Glu Gly Ile Val Leu Glu Lys Ala Pro Met

145                 150                 155                 160

Pro Gln Val Ala His Phe Arg Asp Pro Arg Val Trp Lys Glu Asn Asn
                165                 170                 175

His Trp Phe Met Val Val Gly Tyr Arg Thr Asp Asp Glu Lys His Gln
            180                 185                 190

Gly Ile Gly His Val Ala Leu Tyr Arg Ser Glu Asn Leu Lys Asp Trp
        195                 200                 205

Ile Phe Val Lys Thr Leu Leu Gly Asp Asn Ser Gln Leu Pro Leu Gly
    210                 215                 220

Lys Arg Ala Phe Met Trp Glu Cys Pro Asp Phe Phe Ser Leu Gly Asn
225                 230                 235                 240

Arg Ser Val Leu Met Phe Ser Pro Gln Gly Leu Lys Ala Ser Gly Tyr
                245                 250                 255

Lys Asn Arg Asn Leu Phe Gln Asn Gly Tyr Ile Leu Gly Lys Trp Gln
            260                 265                 270

Ala Pro Gln Phe Thr Pro Glu Thr Ser Phe Gln Glu Leu Asp Tyr Gly
        275                 280                 285

His Asp Phe Tyr Ala Ala Gln Arg Phe Glu Ala Lys Asp Gly Arg Gln
    290                 295                 300

Ile Leu Ile Ala Trp Phe Asp Met Trp Glu Asn Gln Lys Pro Ser Gln
305                 310                 315                 320

Arg Asp Gly Trp Ala Gly Cys Met Thr Leu Pro Arg Lys Leu Asp Leu
                325                 330                 335

Ile Asp Asn Lys Ile Val Met Thr Pro Val Arg Glu Met Glu Ile Leu
            340                 345                 350

Arg Gln Ser Glu Lys Ile Glu Ser Val Val Thr Leu Ser Asp Ala Glu
        355                 360                 365

His Pro Phe Thr Met Asp Ser Pro Leu Gln Glu Ile Glu Leu Ile Phe
    370                 375                 380

Asp Leu Glu Lys Ser Ser Ala Tyr Gln Ala Gly Leu Ala Leu Arg Cys
385                 390                 395                 400

Asn Gly Lys Gly Gln Glu Thr Leu Leu Tyr Ile Asp Arg Ser Gln Asn
                405                 410                 415

Arg Ile Ile Leu Asp Arg Asn Arg Ser Gly Gln Asn Val Lys Gly Ile
            420                 425                 430

Arg Ser Cys Pro Leu Pro Asn Thr Ser Lys Val Arg Leu His Ile Phe
        435                 440                 445

Leu Asp Arg Ser Ser Ile Glu Ile Phe Val Gly Asp Asp Gln Thr Gln
    450                 455                 460

Gly Leu Tyr Ser Ile Ser Ser Arg Ile Phe Pro Asp Lys Asp Ser Leu
465                 470                 475                 480

Lys Gly Arg Leu Phe Ala Ile Glu Gly Tyr Ala Val Phe Asp Ser Phe
                485                 490                 495

Lys Arg Trp Thr Leu Gln Asp Ala Asn Leu Ala Ala Phe Ser Ser Asp
            500                 505                 510

Ala Cys Gly Ser Ser His His His His His His
        515                 520

<210> 3
<211> 7151
<212> DNA
<213> Artificial Sequence

<220>
<223> Description of Artificial Sequence: Synthetic
      polynucleotide

<400> 3
agcttgtcat ctgccggatg aggcaaaacc ctgcctacgg cgcgattaca tcgtcccagc      60

gcgatcgctc ttactgttga tggctcgtgc ttaaaaacaa tgcaaacttc accgtttcag     120

ctggtgattt tcgactgtga tggtgtgctt gttgatagcg gaacgcatca ctaatcgcgt     180

ctttgcagac atgctcaatg aactgggtct gttggtgact ttggatgaca tgtttgagca     240

gtttgtgggt cattccatgg ctgactgtct caaactaatt gagcgacggt taggcaatcc     300

tccaccccct gactttgttc agcactatca acgccgtacc cgtatcgcgt tagaaacgca     360

tctacaagcc gttcctgggg ttgaagaggc tttggatgct cttgaattgc cctactgtgt     420

tgcgtccagt ggtgatcatc aaaagatgcg aaccacactg agcctgacga agctctggcc     480

acgatttgag ggacgaatct tcagcgtgac tgaagtacct cgcggcaagc catttcccga     540

tgtctttttg ttggccgccg atcgcttcgg ggttaatcct acggcctgcg ctgtgatcga     600

agacaccccc ttgggagtag cggcaggcgt ggcggcagga atgcaagtgt ttggctacgc     660

gggttccatg cccgcttggc gtctgcaaga agccggtgcc catctcattt ttgacgatat 720

gcgactgctg cccagtctgc tccaatcgtc gccaaaagat aactccacag cattgcccaa 780

tccctaaccc ctgctcgcgc cgcaactaca cactaaaccg ttcctgcgcg atcgctctta 840

ctgttgatgg ctcgtgctta aaaacaatgc aaccctaacc gtttcagctg gtgattttcg 900

gacgatttgg cttacaggga taactgagag tcaacagcct ctgtccgtca ttgcacaccc 960

atccatgcac tggggacttg actcatgctg aatcacattt cccttgtcca ttgggcgaga 1020

ggggagggga atcttctgga ctcttcacta agcggcgatc gcaggttctt ctacccaagc 1080

agtggcgatc gcttgattgc agtcttcaat gctggcctct gcagccatcg ccgccaccaa 1140

agcatcgtag gcgggacgtt gttgctccag taaagtcttc gcccgtaaca atccccagcg 1200

actgcgtaaa tccgcttcgg caggattgcg atcgagttgc cgccacagtt gtttccactg 1260

ggcgcgatcg tcagctcccc cttccacgtt gccgtagacc agttgctctg ccgctgcacc 1320

ggccatcaac acctgacacc actgttccag cgatcgctga ctgagttgcc cctgtgcggc 1380

ttcggcttct agcgcagctg cttggaactg cacacccccg cgaccaggtt gtccttggcg 1440

cagcgcttcc cacgctgaga gggtgtagcc cgtcacgggt aaccgatatc tgtacaccgc 1500

ggggtaccac tagttacaga caagctgtga ccgtctccgg gagctgcatg tgtcagaggt 1560

tttcaccgtc atcaccgaaa cgcgcgaggc agcagatcaa ttcgcgcgcg aaggcgaagc 1620

ggcatgcatt tacgttgaca ccatcgaatg gtgcaaaacc tttcgcggta tggcatgata 1680

gcgcccggaa gagagtcaat tcagggtggt gaatgtgaaa ccagtaacgt tatacgatgt 1740

cgcagagtat gccggtgtct cttatcagac cgtttcccgc gtggtgaacc aggccagcca 1800

cgtttctgcg aaaacgcggg aaaaagtgga agcggcgatg gcggagctga attacattcc 1860

caaccgcgtg gcacaacaac tggcgggcaa acagtcgttg ctgattggcg ttgccacctc 1920

cagtctggcc ctgcacgcgc cgtcgcaaat tgtcgcggcg attaaatctc gcgccgatca 1980

actgggtgcc agcgtggtgg tgtcgatggt agaacgaagc ggcgtcgaag cctgtaaagc 2040

ggcggtgcac aatcttctcg cgcaacgcgt cagtgggctg atcattaact atccgctgga 2100

tgaccaggat gccattgctg tggaagctgc ctgcactaat gttccggcgt tatttcttga 2160

tgtctctgac cagacaccca tcaacagtat tattttctcc catgaagacg gtacgcgact 2220

gggcgtggag catctggtcg cattgggtca ccagcaaatc gcgctgttag cgggcccatt 2280

aagttctgtc tcggcgcgtc tgcgtctggc tggctggcat aaatatctca ctcgcaatca 2340

aattcagccg atagcggaac gggaaggcga ctggagtgcc atgtccggtt ttcaacaaac 2400

catgcaaatg ctgaatgagg gcatcgttcc cactgcgatg ctggttgcca acgatcagat 2460

ggcgctgggc gcaatgcgcg ccattaccga gtccgggctg cgcgttggtg cggatatctc 2520

ggtagtggga tacgacgata ccgaagacag ctcatgttat atcccgccgt taaccaccat 2580

caaacaggat tttcgcctgc tggggcaaac cagcgtggac cgcttgctgc aactctctca 2640

gggccaggcg gtgaagggca atcagctgtt gcccgtctca ctggtgaaaa gaaaaaccac 2700

cctggcgccc aatacgcaaa ccgcctctcc ccgcgcgttg gccgattcat taatgcagct 2760

ggcacgacag gtttcccgac tggaaagcgg gcagtgagcg caacgcaatt aatgtgagtt 2820

agcgcgaatt gatctggttt gacagcttat catcgactgc acggtgcacc aatgcttctg 2880

gcgtcaggca gccatcggaa gctgtggtat ggctgtgcag gtcgtaaatc actgcataat 2940

tcgtgtcgct caaggcgcac tcccgttctg gataatgttt tttgcgccga catcataacg 3000

gttctggcaa atattctgaa atgagctgtt gacaattaat catccggctc gtataatgtg 3060

tggaattgtg agcggataac aatttcacac aaggaggaaa aaaaaatgtc tagtgaaagc 3120

agccagggcc tggtgacccg cctggccctg atcgctgcta tcggtggtct gctgtttggc 3180

tacgatagcg ccgtgatcgc cgccatcggt accccagtgg atatccactt tatcgctccc 3240

cgccacctga gcgctaccgc cgccgccagc ctgagcggta tggtggtggt ggctgtgctg 3300

gtcggctgcg tgaccggcag cctgctgagc ggttggatcg gcatccgctt tggccgccgc 3360

ggtggtctgc tgatgagcag catctgcttt gtggccgccg gctttggtgc tgccctgacc 3420

gaaaaactgt ttggtaccgg tggtagcgct ctgcaaatct tttgcttttt ccgctttctg 3480

gccggcctgg gcatcggcgt cgtgagcacc ctgaccccca cctacatcgc cgaaatcgcc 3540

ccccccgata aacgcggtca gatggtcagc ggccagcaga tggctatcgt gaccggtgcc 3600

ctgaccggtt acatctttac ctggctgctg gcccactttg gcagcatcga ttgggtgaac 3660

gccagcggtt ggtgctggag cccagccagc gaaggtctga tcggtatcgc ctttctgctg 3720

ctgctgctga ccgctccaga taccccccac tggctggtga tgaaaggccg ccacagcgaa 3780

gccagcaaaa tcctggcccg cctggaaccc caggccgatc ccaacctgac catccagaaa 3840

atcaaagccg gctttgataa agccatggat aaaagcagcg ccggcctgtt tgcctttggc 3900

atcaccgtgg tgtttgccgg cgtgagcgtg gccgcctttc agcagctggt cggcatcaac 3960

gccgtgctgt actacgcccc ccagatgttt cagaacctgg gctttggcgc cgataccgcc 4020

ctgctgcaaa ccatcagcat cggcgtggtg aactttatct ttaccatgat cgccagccgc 4080

gtggtggatc gctttggtcg caaaccactg ctgatctggg gtgccctggg catggctgct 4140

atgatggctg tgctgggctg ctgcttttgg tttaaagtgg gcggcgtgct gcccctggcc 4200

agcgtgctgc tgtacatcgc cgtgtttggc atgagctggg gccccgtgtg ctgggtggtg 4260

ctgagcgaaa tgtttcccag cagcatcaaa ggcgccgcca tgcccatcgc cgtgaccggc 4320

cagtggctgg ccaacatcct ggtgaacttt ctgtttaaag tggccgatgg cagccccgcc 4380

ctgaaccaga cctttaacca cggctttagc tacctggtgt ttgccgccct gagcatcctg 4440

ggcggcctga tcgtggcccg ctttgtgccc gaaaccaaag gccgcagcct ggatgaaatc 4500

gaagaaatgt ggcgcagcca gaaaggcagc agccaccacc accaccacca ctaatctaga 4560

aagcttgcgg ccgcgctagc aagcttggcc ggatccggcc ggatccggga gtttgtagaa 4620

acgcaaaaag gccatccgtc aggatggcct tctgcttaat ttgatgcctg gcagtttatg 4680

gcgggcgtcc tgcccgccac cctccgggcc gttgcttcgc aacgttcaaa tccgctcccg 4740

gcggatttgt cctactcagg agagcgttca ccgacaaaca acagataaaa cgaaaggccc 4800

agtctttcga ctgagccttt cgttttattt gatgcctggc agttccctac tctcgcatgg 4860

ggagacccca cactaccatc ggcgctacgg cgtttcactt ctgagttcgg catggggtca 4920

ggtgggacca ccgcgctact gccgccaggc aaattctgtt ttattgagcc gttaccccac 4980

ctactagcta atcccatctg ggcacatccg atggcaagag gcccgaaggt ccccctcttt 5040

ggtcttgcga cgttatgcgg tattagctac cgtttccagt agttatcccc ctccatcagg 5100

cagtttccca gacattactc acccgtccgc cactcgtcag caaagaagca agcttagatc 5160

gacctgcagg gggggggggg aaagccacgt tgtgtctcaa aatctctgat gttacattgc 5220

acaagataaa aatatatcat catgaacaat aaaactgtct gcttacataa acagtaatac 5280

aaggggtgtt atgagccata ttcaacggga aacgtcttgc tcgaggccgc gattaaattc 5340

caacatggat gctgatttat atgggtataa atgggctcgc gataatgtcg ggcaatcagg 5400

tgcgacaatc tatcgattgt atgggaagcc cgatgcgcca gagttgtttc tgaaacatgg 5460

caaaggtagc gttgccaatg atgttacaga tgagatggtc agactaaact ggctgacgga 5520

atttatgcct cttccgacca tcaagcattt tatccgtact cctgatgatg catggttact 5580

caccactgcg atccccggga aaacagcatt ccaggtatta gaagaatatc ctgattcagg 5640

tgaaaatatt gttgatgcgc tggcagtgtt cctgcgccgg ttgcattcga ttcctgtttg 5700

taattgtcct tttaacagcg atcgcgtatt tcgtctcgct caggcgcaat cacgaatgaa 5760

taacggtttg gttgatgcga gtgattttga tgacgagcgt aatggctggc ctgttgaaca 5820

agtctggaaa gaaatgcata agcttttgcc attctcaccg gattcagtcg tcactcatgg 5880

tgatttctca cttgataacc ttatttttga cgaggggaaa ttaataggtt gtattgatgt 5940

tggacgagtc ggaatcgcag accgatacca ggatcttgcc atcctatgga actgcctcgg 6000

tgagttttct ccttcattac agaaacggct ttttcaaaaa tatggtattg ataatcctga 6060

tatgaataaa ttgcagtttc atttgatgct cgatgagttt ttctaatcag aattggttaa 6120

ttggttgtaa cactggcaga gcattacgct gacttgacgg gacggcggct ttgttgaata 6180

aatcgaactt ttgctgagtt gaaggatcag atcacgcatc ttcccgacaa cgcagaccgt 6240

tccgtggcaa agcaaaagtt caaaatcacc aactggtcca cctacaacaa agctctcatc 6300

aaccgtggct ccctcacttt ctggctggat gatggggcga ttcaggcctg gtatgagtca 6360

gcaacacctt cttcacgagg cagacctcag cgcccccccc cccctgcagg tcgatctggt 6420

aaccccagcg cggttgctac caagtagtga cccgcttcgt gatgcaaaat ccgctgacga 6480

tattcgggcg atcgctgctg aatgccatcg agcagtaacg tggcaccccg cccctgccaa 6540

gtcaccgcat ccagactgaa cagcaccaag aggctaaaac ccaatcccgc cggtagcagc 6600

ggagaactac ccagcattgg tcccaccaaa gctaatgccg tcgtggtaaa aatcgcgatc 6660

gccgtcagac tcaagcccag ttcgctcatg cttcctcatc taggtcacag tcttcggcga 6720

tcgcatcgat ctgatgctgc agcaagcgtt tttccatacg gcgatcgcgc cgtcgccctt 6780

tcgctgccgt ggcccgctta cgagctcgtt tatcgaccac gatcgcatcc aaatccgcga 6840

tcgcttccca gtccggcaat tcagtctggg gcgtccgttt cattaatcct gatcaggcac 6900

gaaattgctg tgcgtagtat cgcgcatagc ggccagcctc tgccaacagc gcatcgtgat 6960

tgcctgcctc aacaatctgg ccgcgctcca tcaccaagat gcggctggca ttacgaaccg 7020

tagccagacg gtgagcaatg ataaagaccg tccgtccctg catcacccgt tctagggcct 7080

cttgcaccaa ggtttcggac tcggaatcaa gcgccgaagt cgcctcatcc agaattaaaa 7140

tgcgtggatc c 7151

<210> 4
<211> 482
<212> PRT
<213> Artificial Sequence

<220>
<223> Description of Artificial Sequence: Synthetic
      polypeptide

<400> 4
Met Ser Ser Glu Ser Ser Gln Gly Leu Val Thr Arg Leu Ala Leu Ile
1               5                   10                  15

Ala Ala Ile Gly Gly Leu Leu Phe Gly Tyr Asp Ser Ala Val Ile Ala
            20                  25                  30

Ala Ile Gly Thr Pro Val Asp Ile His Phe Ile Ala Pro Arg His Leu
        35                  40                  45

Ser Ala Thr Ala Ala Ala Ser Leu Ser Gly Met Val Val Val Ala Val
    50                  55                  60

Leu Val Gly Cys Val Thr Gly Ser Leu Leu Ser Gly Trp Ile Gly Ile
65                  70                  75                  80

Arg Phe Gly Arg Arg Gly Gly Leu Leu Met Ser Ser Ile Cys Phe Val
                85                  90                  95

Ala Ala Gly Phe Gly Ala Ala Leu Thr Glu Lys Leu Phe Gly Thr Gly
            100                 105                 110

Gly Ser Ala Leu Gln Ile Phe Cys Phe Phe Arg Phe Leu Ala Gly Leu
        115                 120                 125

Gly Ile Gly Val Val Ser Thr Leu Thr Pro Thr Tyr Ile Ala Glu Ile
    130                 135                 140

Ala Pro Pro Asp Lys Arg Gly Gln Met Val Ser Gly Gln Gln Met Ala
145                 150                 155                 160

Ile Val Thr Gly Ala Leu Thr Gly Tyr Ile Phe Thr Trp Leu Leu Ala
                165                 170                 175

His Phe Gly Ser Ile Asp Trp Val Asn Ala Ser Gly Trp Cys Trp Ser
            180                 185                 190

Pro Ala Ser Glu Gly Leu Ile Gly Ile Ala Phe Leu Leu Leu Leu Leu
        195                 200                 205

Thr Ala Pro Asp Thr Pro His Trp Leu Val Met Lys Gly Arg His Ser
    210                 215                 220

Glu Ala Ser Lys Ile Leu Ala Arg Leu Glu Pro Gln Ala Asp Pro Asn
225                 230                 235                 240

Leu Thr Ile Gln Lys Ile Lys Ala Gly Phe Asp Lys Ala Met Asp Lys
                245                 250                 255

Ser Ser Ala Gly Leu Phe Ala Phe Gly Ile Thr Val Val Phe Ala Gly
            260                 265                 270

Val Ser Val Ala Ala Phe Gln Gln Leu Val Gly Ile Asn Ala Val Leu
        275                 280                 285

Tyr Tyr Ala Pro Gln Met Phe Gln Asn Leu Gly Phe Gly Ala Asp Thr
    290                 295                 300

Ala Leu Leu Gln Thr Ile Ser Ile Gly Val Val Asn Phe Ile Phe Thr
305                 310                 315                 320

Met Ile Ala Ser Arg Val Val Asp Arg Phe Gly Arg Lys Pro Leu Leu
                325                 330                 335

Ile Trp Gly Ala Leu Gly Met Ala Ala Met Met Ala Val Leu Gly Cys

340                 345                 350

Cys Phe Trp Phe Lys Val Gly Gly Val Leu Pro Leu Ala Ser Val Leu
        355                 360                 365

Leu Tyr Ile Ala Val Phe Gly Met Ser Trp Gly Pro Val Cys Trp Val
    370                 375                 380

Val Leu Ser Glu Met Phe Pro Ser Ser Ile Lys Gly Ala Ala Met Pro
385                 390                 395                 400

Ile Ala Val Thr Gly Gln Trp Leu Ala Asn Ile Leu Val Asn Phe Leu
                405                 410                 415

Phe Lys Val Ala Asp Gly Ser Pro Ala Leu Asn Gln Thr Phe Asn His
            420                 425                 430

Gly Phe Ser Tyr Leu Val Phe Ala Ala Leu Ser Ile Leu Gly Gly Leu
        435                 440                 445

Ile Val Ala Arg Phe Val Pro Glu Thr Lys Gly Arg Ser Leu Asp Glu
    450                 455                 460

Ile Glu Glu Met Trp Arg Ser Gln Lys Gly Ser Ser His His His His
465                 470                 475                 480

His His

<210> 5
<211> 1044
<212> DNA
<213> Artificial Sequence

<220>
<223> Description of Artificial Sequence: Synthetic
      polynucleotide

<400> 5
aaggaggaaa aaaaaatgtc tagacatcac catcatcatc accctaggaa actcgccgtt      60

tatagcacaa aacagtacga caagaagtac ctgcaacagg tgaacgagtc ctttggcttt     120

gagctggaat tttttgactt tctgctgacg gaaaaaaccg ctaaaactgc caatggctgc     180

gaagcggtat gtattttcgt aaacgatgac ggcagccgcc cggtgctgga agagctgaaa     240

aagcacggcg ttaaatatat cgccctgcgc tgtgccggtt tcaataacgt cgaccttgac     300

gcggcaaaag aactggggct gaaagtagtc cgtgttccag cctatgatcc agaggccgtt     360

gctgaacacg ccatcggtat gatgatgacg ctgaaccgcc gtattcaccg cgcgtatcag     420

cgtacccgtg atgctaactt ctctctggaa ggtctgaccg gctttactat gtatggcaaa 480

acggcaggcg ttatcggtac cggtaaaatc ggtgtggcga tgctgcgcat tctgaaaggt 540

tttggtatgc gtctgctggc gttcgatccg tatccaagtg cagcggcgct ggaactcggt 600

gtggagtatg tcgatctgcc aaccctgttc tctgaatcag acgttatctc tctgcactgc 660

ccgctgacac cggaaaacta tcatctgttg aacgaagccg ccttcgaaca gatgaaaaat 720

ggcgtgatga tcgtcaatac cagtcgcggt gcattgattg attctcaggc agcaattgaa 780

gcgctgaaaa atcagaaaat tggttcgttg ggtatggacg tgtatgagaa cgaacgcgat 840

ctattctttg aagataaatc caacgacgtg atccaggatg acgtattccg tcgcctgtct 900

gcctgccaca acgtgctgtt taccgggcac caggcattcc tgacagcaga agctctgacc 960

agtatttctc agactacgct gcaaaactta agcaatctgg aaaaaggcga aacctgcccg 1020

aacgaactgg tttaagcggc cgcg 1044

<210> 6
<211> 338
<212> PRT
<213> Artificial Sequence

<220>
<223> Description of Artificial Sequence: Synthetic
      polypeptide

<400> 6
Met Ser Arg His His His His His Pro Arg Lys Leu Ala Val Tyr Ser
1               5                   10                  15

Thr Lys Gln Tyr Asp Lys Lys Tyr Leu Gln Gln Val Asn Glu Ser Phe
            20                  25                  30

Gly Phe Glu Leu Glu Phe Phe Asp Phe Leu Leu Thr Glu Lys Thr Ala
        35                  40                  45

Lys Thr Ala Asn Gly Cys Glu Ala Val Cys Ile Phe Val Asn Asp Asp
    50                  55                  60

Gly Ser Arg Pro Val Leu Glu Glu Leu Lys Lys His Gly Val Lys Tyr
65                  70                  75                  80

Ile Ala Leu Arg Cys Ala Gly Phe Asn Asn Val Asp Leu Asp Ala Ala
                85                  90                  95

Lys Glu Leu Gly Leu Lys Val Val Arg Val Pro Ala Tyr Asp Pro Glu
            100                 105                 110

Ala Val Ala Glu His Ala Ile Gly Met Met Met Thr Leu Asn Arg Arg
        115                 120                 125

Ile His Arg Ala Tyr Gln Arg Thr Arg Asp Ala Asn Phe Ser Leu Glu
    130                 135                 140

Gly Leu Thr Gly Phe Thr Met Tyr Gly Lys Thr Ala Gly Val Ile Gly
145                 150                 155                 160

Thr Gly Lys Ile Gly Val Ala Met Leu Arg Ile Leu Lys Gly Phe Gly
                165                 170                 175

Met Arg Leu Leu Ala Phe Asp Pro Tyr Pro Ser Ala Ala Ala Leu Glu
            180                 185                 190

Leu Gly Val Glu Tyr Val Asp Leu Pro Thr Leu Phe Ser Glu Ser Asp
        195                 200                 205

Val Ile Ser Leu His Cys Pro Leu Thr Pro Glu Asn Tyr His Leu Leu
    210                 215                 220

Asn Glu Ala Ala Phe Glu Gln Met Lys Asn Gly Val Met Ile Val Asn
225                 230                 235                 240

Thr Ser Arg Gly Ala Leu Ile Asp Ser Gln Ala Ala Ile Glu Ala Leu
                245                 250                 255

Lys Asn Gln Lys Ile Gly Ser Leu Gly Met Asp Val Tyr Glu Asn Glu
            260                 265                 270

Arg Asp Leu Phe Phe Glu Asp Lys Ser Asn Asp Val Ile Gln Asp Asp
        275                 280                 285

Val Phe Arg Arg Leu Ser Ala Cys His Asn Val Leu Phe Thr Gly His
    290                 295                 300

Gln Ala Phe Leu Thr Ala Glu Ala Leu Thr Ser Ile Ser Gln Thr Thr
305                 310                 315                 320

Leu Gln Asn Leu Ser Asn Leu Glu Lys Gly Glu Thr Cys Pro Asn Glu
                325                 330                 335

Leu Val

<210> 7
<211> 1718
<212> DNA
<213> Artificial Sequence

<220>
<223> Description of Artificial Sequence: Synthetic
      polynucleotide

<400> 7
ggaggaaaaa aaaatgtcta gaaagcttgc ggccgcacat caccatcatc atcacaatct      60

ctggcaacaa aactacgatc ccgccgggaa tatctggctt tccagtctga tagcatcgct     120

tcccatcctg tttttcttct ttgcgctgat taagctcaaa ctgaaaggat acgtcgccgc     180

ctcgtggacg gtggcaatcg cccttgccgt ggctttgctg ttctataaaa tgccggtcgc     240

taacgcgctg gcctcggtgg tttatggttt cttctacggg ttgtggccca tcgcgtggat     300

cattattgca gcggtgttcg tctataagat ctcggtgaaa accgggcagt ttgacatcat     360

tcgctcgtct attctttcga taacccctga ccagcgtctg caaatgctga tcgtcggttt     420

ctgtttcggc gcgttccttg aaggagccgc aggctttggc gcaccggtag caattaccgc     480

cgcattgctg gtcggcctgg gttttaaacc gctgtacgcc gccgggctgt gcctgattgt     540

taacaccgcg ccagtggcat ttggtgcgat gggcattcca atcctggttg ccggacaggt     600

aacaggtatc gacagctttg agattggtca gatggtgggg cggcagctac cgtttatgac     660

cattatcgtg ctgttctgga tcatggcgat tatggacggc tggcgcggta tcaaagagac     720

gtggcctgcg gtcgtggttg cgggcggctc gtttgccatc gctcagtacc ttagctctaa     780

cttcattggg ccggagctgc cggacattat ctcttcgctg gtatcactgc tctgcctgac     840

gctgttcctc aaacgctggc agccagtgcg tgtattccgt tttggtgatt tgggggcgtc     900

acaggttgat atgacgctgg cccacaccgg ttacactgcg ggtcaggtgt tacgtgcctg     960

gacaccgttc ctgttcctga cagctaccgt aacactgtgg agtatcccgc cgtttaaagc    1020

cctgttcgca tcgggtggcg cgctgtatga gtgggtgatc aatattccgg tgccgtacct    1080

cgataaactg gttgcccgta tgccgccagt ggtcagcgag gctacagcct atgccgccgt    1140

gtttaagttt gactggttct ctgccaccgg caccgccatt ctgtttgctg cactgctctc    1200

gattgtctgg ctgaagatga aaccgtctga cgctatcagc accttcggca gcacgctgaa    1260

agaactggct ctgcccatct actccatcgg tatggtgctg gcattcgcct ttatttcgaa    1320

ctattccgga ctgtcatcaa cactggcgct ggcactggcg cacaccggtc atgcattcac    1380

cttcttctcg ccgttcctcg gctggctggg ggtattcctg accgggtcgg atacctcatc    1440

taacgccctg ttcgccgcgc tgcaagccac cgcagcacaa caaattggcg tctctgatct    1500

gttgctggtt gccgccaata ccaccggtgg cgtcaccggt aagatgatct ccccgcaatc    1560

tatcgctatc gcctgtgcgg cggtaggcct ggtgggcaaa gagtctgatt tgttccgctt    1620

tactgtcaaa cacagcctga tcttcacctg tatagtgggc gtgatcacca cgcttcaggc    1680

ttatgtctta acgtggatga ttccttaagc ggccgccg                            1718

<210> 8
<211> 564
<212> PRT
<213> Artificial Sequence

<220>
<223> Description of Artificial Sequence: Synthetic
      polypeptide

<400> 8
Met Ser Arg Lys Leu Ala Ala Ala His His His His His His Asn Leu
1               5                   10                  15

Trp Gln Gln Asn Tyr Asp Pro Ala Gly Asn Ile Trp Leu Ser Ser Leu
            20                  25                  30

Ile Ala Ser Leu Pro Ile Leu Phe Phe Phe Phe Ala Leu Ile Lys Leu
        35                  40                  45

Lys Leu Lys Gly Tyr Val Ala Ala Ser Trp Thr Val Ala Ile Ala Leu
    50                  55                  60

Ala Val Ala Leu Leu Phe Tyr Lys Met Pro Val Ala Asn Ala Leu Ala
65                  70                  75                  80

Ser Val Val Tyr Gly Phe Phe Tyr Gly Leu Trp Pro Ile Ala Trp Ile
                85                  90                  95

Ile Ile Ala Ala Val Phe Val Tyr Lys Ile Ser Val Lys Thr Gly Gln
            100                 105                 110

Phe Asp Ile Ile Arg Ser Ser Ile Leu Ser Ile Thr Pro Asp Gln Arg
        115                 120                 125

Leu Gln Met Leu Ile Val Gly Phe Cys Phe Gly Ala Phe Leu Glu Gly
    130                 135                 140

Ala Ala Gly Phe Gly Ala Pro Val Ala Ile Thr Ala Ala Leu Leu Val
145                 150                 155                 160

Gly Leu Gly Phe Lys Pro Leu Tyr Ala Ala Gly Leu Cys Leu Ile Val
                165                 170                 175

Asn Thr Ala Pro Val Ala Phe Gly Ala Met Gly Ile Pro Ile Leu Val
            180                 185                 190

Ala Gly Gln Val Thr Gly Ile Asp Ser Phe Glu Ile Gly Gln Met Val
        195                 200                 205

Gly Arg Gln Leu Pro Phe Met Thr Ile Ile Val Leu Phe Trp Ile Met
    210                 215                 220

Ala Ile Met Asp Gly Trp Arg Gly Ile Lys Glu Thr Trp Pro Ala Val
225                 230                 235                 240

Val Val Ala Gly Gly Ser Phe Ala Ile Ala Gln Tyr Leu Ser Ser Asn
                245                 250                 255

Phe Ile Gly Pro Glu Leu Pro Asp Ile Ile Ser Ser Leu Val Ser Leu
            260                 265                 270

Leu Cys Leu Thr Leu Phe Leu Lys Arg Trp Gln Pro Val Arg Val Phe
        275                 280                 285

Arg Phe Gly Asp Leu Gly Ala Ser Gln Val Asp Met Thr Leu Ala His
    290                 295                 300

Thr Gly Tyr Thr Ala Gly Gln Val Leu Arg Ala Trp Thr Pro Phe Leu
305                 310                 315                 320

Phe Leu Thr Ala Thr Val Thr Leu Trp Ser Ile Pro Pro Phe Lys Ala
                325                 330                 335

Leu Phe Ala Ser Gly Gly Ala Leu Tyr Glu Trp Val Ile Asn Ile Pro
            340                 345                 350

Val Pro Tyr Leu Asp Lys Leu Val Ala Arg Met Pro Pro Val Val Ser
        355                 360                 365

Glu Ala Thr Ala Tyr Ala Ala Val Phe Lys Phe Asp Trp Phe Ser Ala
    370                 375                 380

Thr Gly Thr Ala Ile Leu Phe Ala Ala Leu Leu Ser Ile Val Trp Leu
385                 390                 395                 400

Lys Met Lys Pro Ser Asp Ala Ile Ser Thr Phe Gly Ser Thr Leu Lys
                405                 410                 415

Glu Leu Ala Leu Pro Ile Tyr Ser Ile Gly Met Val Leu Ala Phe Ala
            420                 425                 430

Phe Ile Ser Asn Tyr Ser Gly Leu Ser Ser Thr Leu Ala Leu Ala Leu
        435                 440                 445

Ala His Thr Gly His Ala Phe Thr Phe Phe Ser Pro Phe Leu Gly Trp
    450                 455                 460

Leu Gly Val Phe Leu Thr Gly Ser Asp Thr Ser Ser Asn Ala Leu Phe
465                 470                 475                 480

Ala Ala Leu Gln Ala Thr Ala Ala Gln Gln Ile Gly Val Ser Asp Leu
                485                 490                 495

Leu Leu Val Ala Ala Asn Thr Thr Gly Gly Val Thr Gly Lys Met Ile
            500                 505                 510

Ser Pro Gln Ser Ile Ala Ile Ala Cys Ala Ala Val Gly Leu Val Gly
        515                 520                 525

Lys Glu Ser Asp Leu Phe Arg Phe Thr Val Lys His Ser Leu Ile Phe
    530                 535                 540

Thr Cys Ile Val Gly Val Ile Thr Thr Leu Gln Ala Tyr Val Leu Thr
545                 550                 555                 560

Trp Met Ile Pro

<210> 9
<211> 4885
<212> DNA
<213> Artificial Sequence

<220>
<223> Description of Artificial Sequence: Synthetic
      polynucleotide

<400> 9
ctagtcgcgt tgctggcgtt tttccatagg ctccgccccc ctgacgagca tcacaaaaat      60

cgacgctcaa gtcagaggtg gcgaaacccg acaggactat aaagatacca ggcgtttccc     120

cctggaagct ccctcgtgcg ctctcctgtt ccgaccctgc cgcttaccgg atacctgtcc     180

gcctttctcc cttcgggaag cgtggcgctt tctcatagct cacgctgtag gtatctcagt     240

tcggtgtagg tcgttcgctc caagctgggc tgtgtgcacg aaccccccgt tcagcccgac     300

cgctgcgcct tatccggtaa ctatcgtctt gagtccaacc cggtaagaca cgacttatcg     360

ccactggcag cagccactgg taacaggatt agcagagcga ggtatgtagg cggtgctaca     420

gagttcttga agtggtggcc taactacggc tacactagaa gaacagtatt tggtatctgc     480

gctctgctga agccagttac cttcggaaaa agagttggta gctcttgatc cggcaaacaa     540

accaccgctg gtagcggtgg tttttttgtt tgcaagcagc agattacgcg cagaaaaaaa     600

ggatctcaag aagatccttt gatcttttct acggggtctg acgctcagtc tagttcagcc     660

agctcgtcgt gatgtcgaaa cccaagccac cctcagaggt gaaggccgct tcgagcacat     720

cgggaagcgt gtcgacgacg gtgctcggtg cctcgggtaa gagccagata gatgcggtga 780

cgttcaccgt cgcgatcgtg gcgggaacca cggtcactgc gtccgttaca actcgcacgt 840

cgtcggccag cacgactgac tcgacggctt cgattagccc gggagaggca aggccatcag 900

gctcggtaga cagaatgctg attaacacct cgccgggagc agggctgctc acagccgcgt 960

ccttcacccg tgggtcagcc gtcagcgctt gatagcggta ccaggccgca ccgcctgcgg 1020

tcgagctgcc cttgatccgc tcgatggtgc gatcgcgaag ctcgccatcc ggctcattcg 1080

gctgccgcac cacgccgtag aacgcggcaa ggttgtcgag gtcggggccg tttgagtagc 1140

gaagcagtgt cgcaagaagt gcgtcgttga tccgctgacg caggatcagc tcgcgggctg 1200

cgcagacctc cagcagcttg atgaccgggt cggattcgag gatggcggtg tagctggcgt 1260

cacgcgatcg caggtcgtcg atcaagtcct gcaggatcag ttcaaagtcc agcgcttcga 1320

tgatggtggg cgcgggaatc gtagcaaagt caagaacggt catgagacga ctaagccctc 1380

cagcgtgata cgcctgccct cggggatgta gtagccgatc aggttcagct cgacttgacc 1440

agctgcgctg gctgagacga tgcgaacctt ctccagcttc agccgtggct cccagcgatc 1500

gagcgcttca gctgtggccg ccaccaggtc aacgatgagg gactggttga tcggtctagt 1560

catcaaataa aacgaaaggc tcagtcgaaa gactgggcct ttcgttttat ctgttgtttg 1620

tcggtgaacg ctctcctgag taggacaaat ccgccgggag cggatttgaa cgttgcgaag 1680

caacggcccg gagggtggcg ggcaggacgc ccgccataaa ctgccaggca tcaaattaag 1740

cagaaggcca tcctgacgga tggccttttt gcgtttctac tctagtttcg aattgtgagc 1800

gctcacaatt tcgaaacccc aggctttaca ctttatgctt ccggctcgta taatgtgtgg 1860

aattgtgagc ggataacaat ttcacacaag gaggaaaaac atatgtctag aaagcttgcg 1920

gccgcgtagg atccgatcgg cacgtaagag gttccaactt tcaccataat gaaataagat 1980

cactaccggg cgtatttttt gagttatcga gattttcagg agctaaggaa gctaaaatgg 2040

agaaaaaaat cactggatat accaccgttg atatatccca atggcatcgt aaagaacatt 2100

ttgaggcatt tcagtcagtt gctcaatgta cctataacca gaccgttcag ctggatatta 2160

cggccttttt aaagaccgta aagaaaaata agcacaagtt ttatccggcc tttattcaca 2220

ttcttgcccg cctgatgaat gctcatccgg agttccgtat ggcaatgaaa gacggtgagc 2280

tggtgatatg ggatagtgtt cacccttgtt acaccgtttt ccatgagcaa actgaaacgt 2340

tttcatcgct ctggagtgaa taccacgacg atttccggca gtttctacac atatattcgc 2400

aagatgtggc gtgttacggt gaaaacctgg cctatttccc taaagggttt attgagaata 2460

tgtttttcgt ctcagccaat ccctgggtga gtttcaccag ttttgattta aacgtggcca 2520

atatggacaa cttcttcgcc cccgttttca ctatgggcaa atattatacg caaggcgaca 2580

aggtgctgat gccgctggcg attcaggttc atcatgccgt ctgtgatggc ttccatgtcg 2640

gcagaatgct taatgaatta caacagtact gcgatgagtg gcagggcggg gcgtaatttt 2700

tttaaggcag ttattggtgc ccttgaattc ctactagtcg aagcggcatg catttacgtt 2760

gacaccatcg aatggtgcaa aacctttcgc ggtatggcat gatagcgccc ggaagagagt 2820

caattcaggg tggtgaatgt gaaaccagta acgttatacg atgtcgcaga gtatgccggt 2880

gtctcttatc agaccgtttc ccgcgtggtg aaccaggcca gccacgtttc tgcgaaaacg 2940

cgggaaaaag tggaagcggc gatggcggag ctgaattaca ttcccaaccg cgtggcacaa 3000

caactggcgg gcaaacagtc gttgctgatt ggcgttgcca cctccagtct ggccctgcac 3060

gcgccgtcgc aaattgtcgc ggcgattaaa tctcgcgccg atcaactggg tgccagcgtg 3120

gtggtgtcga tggtagaacg aagcggcgtc gaagcctgta aagcggcggt gcacaatctt 3180

ctcgcgcaac gcgtcagtgg gctgatcatt aactatccgc tggatgacca ggatgccatt 3240

gctgtggaag ctgcctgcac taatgttccg gcgttatttc ttgatgtctc tgaccagaca 3300

cccatcaaca gtattatttt ctcccatgaa gacggtacgc gactgggcgt ggagcatctg 3360

gtcgcattgg gtcaccagca aatcgcgctg ttagcgggcc cattaagttc tgtctcggcg 3420

cgtctgcgtc tggctggctg gcataaatat ctcactcgca atcaaattca gccgatagcg 3480

gaacgggaag gcgactggag tgccatgtcc ggttttcaac aaaccatgca aatgctgaat 3540

gagggcatcg ttcccactgc gatgctggtt gccaacgatc agatggcgct gggcgcaatg 3600

cgcgccatta ccgagtccgg gctgcgcgtt ggtgcggata tctcggtagt gggatacgac 3660

gataccgaag acagctcatg ttatatcccg ccgttaacca ccatcaaaca ggattttcgc 3720

ctgctggggc aaaccagcgt ggaccgcttg ctgcaactct ctcagggcca ggcggtgaag 3780

ggcaatcagc tgttgcccgt ctcactggtg aaaagaaaaa ccaccctggc gcccaatacg 3840

caaaccgcct ctccccgcgc gttggccgat tcattaatgc agctggcacg acaggtttcc 3900

cgactggaaa gcgggcagtg atcccacagc cgccagttcc gctggcggca ttttaacttt 3960

ctttaatgaa tctagtgaca agccggggca gacgtgagcc gtagtcccgt cgccagacgc 4020

gggtgcccac gggcgtcgtc aggatgtccg taattgactg ccggaggtgg tcaatgccct 4080

tcagctcctt gccactgtca cggctcatgc ctcgggtcat tagtcgcccg ctccggtatc 4140

ttcactggct tcgatgattg ccgccccgca gctgcagagg tcaccgatcc gagcagtcgg 4200

cctctggttg gtaaagaccg tgcgactgcc ggtgatgatc gtgttcaagc catgcagggg 4260

gcaggcgtga aggtcatcct ttcgggccac ggggcggctg ttgacgaagg tgtcgtcgct 4320

cccggtgatg atgatcccgc cgtgatcggt cacgtcgttt agtcgagcga tgcctggcgt 4380

cgtagtcacg ggtttaggtc aatacgactt gcggtcactg taacgttgcc ctcggcggtc 4440

acgttaacgt cgccttgggc ttcgacttgc gcctcctgca caaggatcac aatccgtcct 4500

tgggctgcgg tgaggtcgat cttgtactca tgcgcttcgc ggtcgtactg gatgattgag 4560

tcatcctcga actgcgtctt ttggatcgtt tctttgtcct cgatctgggg gtagtcagtc 4620

gagaacgcgc cgggcatcgc gaagccctga ctgatctcgc cggagggggc catcacgacg 4680

acggcctcac cgacctcggg cgcccaccag aaccgatcct tgcccgctcg ctgcgtgagc 4740

cacggaatcc agtcagtgag gagcagcgcc tcgccgctct cctcgtcttc ctcgatcgcg 4800

acacggatca gccccttggg atagtcagcc tcggctaccc tgcctacgcg gagcaagttg 4860

ccgtgacgcc gactgtctcg agtat 4885

<210> 10
<211> 1248
<212> DNA
<213> Escherichia coli

<400> 10
atggcactga atattccatt cagaaatgcg tactatcgtt ttgcatccag ttactcattt 60

ctctttttta tttcctggtc gctgtggtgg tcgttatacg ctatttggct gaaaggacat 120

ctagggttga cagggacgga attaggtaca ctttattcgg tcaaccagtt taccagcatt 180

ctatttatga tgttctacgg catcgttcag gataaactcg gtctgaagaa accgctcatc 240

tggtgtatga gtttcatcct ggtcttgacc ggaccgttta tgatttacgt ttatgaaccg 300

ttactgcaaa gcaatttttc tgtaggtcta attctggggg cgctattttt tggcttgggg 360

tatctggcgg gatgcggttt gcttgatagc ttcaccgaaa aaatggcgcg aaattttcat 420

ttcgaatatg gaacagcgcg cgcctgggga tcttttggct atgctattgg cgcgttcttt 480

gccggcatat tttttagtat cagtccccat atcaacttct ggttggtctc gctatttggc 540

gctgtattta tgatgatcaa catgcgtttt aaagataagg atcaccagtg cgtagcggca 600

gatgcgggag gggtaaaaaa agaggatttt atcgcagttt tcaaggatcg aaacttctgg 660

gttttcgtca tatttattgt ggggacgtgg tctttctata acatttttga tcaacaactt 720

tttcctgtct tttattcagg tttattcgaa tcacacgatg taggaacgcg cctgtatggt 780

tatctcaact cattccaggt ggtactcgaa gcgctgtgca tggcgattat tcctttcttt 840

gtgaatcggg tagggccaaa aaatgcatta cttatcggag ttgtgattat ggcgttgcgt 900

atcctttcct gcgcgctgtt cgttaacccc tggattattt cattagtgaa gttgttacat 960

gccattgagg ttccactttg tgtcatatcc gtcttcaaat acagcgtggc aaactttgat 1020

aagcgcctgt cgtcgacgat ctttctgatt ggttttcaaa ttgccagttc gcttgggatt 1080

gtgctgcttt caacgccgac tgggatactc tttgaccacg caggctacca gacagttttc 1140

ttcgcaattt cgggtattgt ctgcctgatg ttgctatttg gcattttctt cttgagtaaa 1200

aaacgcgagc aaatagttat ggaaacgcct gtaccttcag caatatag 1248

<210> 11
<211> 415
<212> PRT
<213> Escherichia coli

<400> 11
Met Ala Leu Asn Ile Pro Phe Arg Asn Ala Tyr Tyr Arg Phe Ala Ser
1               5                   10                  15

Ser Tyr Ser Phe Leu Phe Phe Ile Ser Trp Ser Leu Trp Trp Ser Leu
            20                  25                  30

Tyr Ala Ile Trp Leu Lys Gly His Leu Gly Leu Thr Gly Thr Glu Leu
        35                  40                  45

Gly Thr Leu Tyr Ser Val Asn Gln Phe Thr Ser Ile Leu Phe Met Met
    50                  55                  60

Phe Tyr Gly Ile Val Gln Asp Lys Leu Gly Leu Lys Lys Pro Leu Ile
65                  70                  75                  80

Trp Cys Met Ser Phe Ile Leu Val Leu Thr Gly Pro Phe Met Ile Tyr
                85                  90                  95

Val Tyr Glu Pro Leu Leu Gln Ser Asn Phe Ser Val Gly Leu Ile Leu
            100                 105                 110

Gly Ala Leu Phe Phe Gly Leu Gly Tyr Leu Ala Gly Cys Gly Leu Leu
        115                 120                 125

Asp Ser Phe Thr Glu Lys Met Ala Arg Asn Phe His Phe Glu Tyr Gly
    130                 135                 140

Thr Ala Arg Ala Trp Gly Ser Phe Gly Tyr Ala Ile Gly Ala Phe Phe
145                 150                 155                 160

Ala Gly Ile Phe Phe Ser Ile Ser Pro His Ile Asn Phe Trp Leu Val
                165                 170                 175

Ser Leu Phe Gly Ala Val Phe Met Met Ile Asn Met Arg Phe Lys Asp
            180                 185                 190

Lys Asp His Gln Cys Val Ala Ala Asp Ala Gly Gly Val Lys Lys Glu
        195                 200                 205

Asp Phe Ile Ala Val Phe Lys Asp Arg Asn Phe Trp Val Phe Val Ile
    210                 215                 220

Phe Ile Val Gly Thr Trp Ser Phe Tyr Asn Ile Phe Asp Gln Gln Leu
225                 230                 235                 240

Phe Pro Val Phe Tyr Ser Gly Leu Phe Glu Ser His Asp Val Gly Thr
                245                 250                 255

Arg Leu Tyr Gly Tyr Leu Asn Ser Phe Gln Val Val Leu Glu Ala Leu
            260                 265                 270

Cys Met Ala Ile Ile Pro Phe Phe Val Asn Arg Val Gly Pro Lys Asn
        275                 280                 285

Ala Leu Leu Ile Gly Val Val Ile Met Ala Leu Arg Ile Leu Ser Cys
    290                 295                 300

Ala Leu Phe Val Asn Pro Trp Ile Ile Ser Leu Val Lys Leu Leu His
305                 310                 315                 320

Ala Ile Glu Val Pro Leu Cys Val Ile Ser Val Phe Lys Tyr Ser Val
                325                 330                 335

Ala Asn Phe Asp Lys Arg Leu Ser Ser Thr Ile Phe Leu Ile Gly Phe
            340                 345                 350

Gln Ile Ala Ser Ser Leu Gly Ile Val Leu Leu Ser Thr Pro Thr Gly
        355                 360                 365

Ile Leu Phe Asp His Ala Gly Tyr Gln Thr Val Phe Phe Ala Ile Ser
    370                 375                 380

Gly Ile Val Cys Leu Met Leu Leu Phe Gly Ile Phe Phe Leu Ser Lys
385                 390                 395                 400

Lys Arg Glu Gln Ile Val Met Glu Thr Pro Val Pro Ser Ala Ile
                405                 410                 415

<210> 12
<211> 1206
<212> DNA
<213> Escherichia coli

<400> 12
atgtttcatc tcgatacttt agcaacgctt gttgccgcaa cgctgacgtt gctgctcggg      60

cgtaagttgg tccattccgt ctcctttttg aagaaataca ccataccgga acctgttgcg     120

ggtggtttgt tggtggcgct ggcgctacta gtactgaaaa aaagcatggg ctgggaagtc     180

aactttgata tgtccctgcg cgatccgtta atgctggctt tcttcgccac cattggcctg     240

aacgccaaca ttgccagttt gcgtgccggt gggcgtgtgg ttggcatctt cttgattgtg     300

gttgttggtc tgttggtgat gcaaaatgcc attggcattg gtatggctag cttgttaggg      360

cttgatccgc tgatggggct gttggccggt tctattacgc tttccggcgg tcacggtacg      420

ggcgctgcgt ggagtaaatt gttcattgaa cgttatggct tcaccaatgc gacggaagtg      480

gcgatggcct gtgcaacgtt cggtctggtg ctgggcggct tgattggcgg tccggtggcg      540

cgctatctgg tgaaacactc caccacgccg aacggtattc cggatgacca ggaagtcccg      600

acggcgtttg aaaagccgga tgtgggacgc atgatcacct cgttggtgct gattgaaact      660

atcgcgctga ttgctatctg cctgacggtg gggaaaattg ttgcgcaact tttggctggc      720

actgcttttg aactgccgac cttcgtctgt gtactgtttg ttggcgtgat tctgagcaac      780

ggtctgtcaa taatgggctt ttaccgcgtc tttgagcgtg cggtatccgt gctgggtaac      840

gtaagcttgt cgttgttcct ggcgatggcg ctgatggggc tgaaactgtg ggagctggct      900

tcgctggcgc tgccgatgct ggcgattctg gtggtacaga ccatcttcat ggcgttgtat      960

gccatcttcg ttacctggcg catgatgggc aaaaactacg atgcggcagt gctggctgcg     1020

ggtcactgtg gttttggcct cggtgcaacg ccaacggcaa tcgccaacat gcaggcgatc     1080

actgaacgct ttggcccgtc gcacatggcg tttttggtgg tgccgatggt cggtgcgttc     1140

tttatcgata tcgtcaatgc gctggtgatt aagctgtatt taatgttgcc gatttttgcc     1200

ggttaa                                                                1206

<210> 13
<211> 401
<212> PRT
<213> Escherichia coli

<400> 13
Met Phe His Leu Asp Thr Leu Ala Thr Leu Val Ala Ala Thr Leu Thr
1               5                   10                  15

Leu Leu Leu Gly Arg Lys Leu Val His Ser Val Ser Phe Leu Lys Lys
            20                  25                  30

Tyr Thr Ile Pro Glu Pro Val Ala Gly Gly Leu Leu Val Ala Leu Ala
        35                  40                  45

Leu Leu Val Leu Lys Lys Ser Met Gly Trp Glu Val Asn Phe Asp Met
    50                  55                  60

Ser Leu Arg Asp Pro Leu Met Leu Ala Phe Phe Ala Thr Ile Gly Leu
65                  70                  75                  80

Asn Ala Asn Ile Ala Ser Leu Arg Ala Gly Gly Arg Val Val Gly Ile
                85                  90                  95

Phe Leu Ile Val Val Val Gly Leu Leu Val Met Gln Asn Ala Ile Gly
            100                 105                 110

Ile Gly Met Ala Ser Leu Leu Gly Leu Asp Pro Leu Met Gly Leu Leu
        115                 120                 125

Ala Gly Ser Ile Thr Leu Ser Gly Gly His Gly Thr Gly Ala Ala Trp
    130                 135                 140

Ser Lys Leu Phe Ile Glu Arg Tyr Gly Phe Thr Asn Ala Thr Glu Val
145                 150                 155                 160

Ala Met Ala Cys Ala Thr Phe Gly Leu Val Leu Gly Gly Leu Ile Gly
                165                 170                 175

Gly Pro Val Ala Arg Tyr Leu Val Lys His Ser Thr Thr Pro Asn Gly
            180                 185                 190

Ile Pro Asp Asp Gln Glu Val Pro Thr Ala Phe Glu Lys Pro Asp Val
        195                 200                 205

Gly Arg Met Ile Thr Ser Leu Val Leu Ile Glu Thr Ile Ala Leu Ile
    210                 215                 220

Ala Ile Cys Leu Thr Val Gly Lys Ile Val Ala Gln Leu Leu Ala Gly
225                 230                 235                 240

Thr Ala Phe Glu Leu Pro Thr Phe Val Cys Val Leu Phe Val Gly Val
                245                 250                 255

Ile Leu Ser Asn Gly Leu Ser Ile Met Gly Phe Tyr Arg Val Phe Glu
            260                 265                 270

Arg Ala Val Ser Val Leu Gly Asn Val Ser Leu Ser Leu Phe Leu Ala
        275                 280                 285

Met Ala Leu Met Gly Leu Lys Leu Trp Glu Leu Ala Ser Leu Ala Leu
    290                 295                 300

Pro Met Leu Ala Ile Leu Val Val Gln Thr Ile Phe Met Ala Leu Tyr
305                 310                 315                 320

Ala Ile Phe Val Thr Trp Arg Met Met Gly Lys Asn Tyr Asp Ala Ala
                325                 330                 335

Val Leu Ala Ala Gly His Cys Gly Phe Gly Leu Gly Ala Thr Pro Thr
            340                 345                 350

Ala Ile Ala Asn Met Gln Ala Ile Thr Glu Arg Phe Gly Pro Ser His
        355                 360                 365

Met Ala Phe Leu Val Val Pro Met Val Gly Ala Phe Phe Ile Asp Ile
    370                 375                 380

Val Asn Ala Leu Val Ile Lys Leu Tyr Leu Met Leu Pro Ile Phe Ala
385                 390                 395                 400

Gly

<210> 14
<211> 6
<212> PRT
<213> Artificial Sequence

<220>
<223> Description of Artificial Sequence: Synthetic
      6xHis tag

<400> 14
His His His His His His
1               5